(19) Europäisches Patentamt
European Patent Office
Office européen des brevets

(11) Publication number: **0 000 978**
**B1**

(12) **EUROPEAN PATENT SPECIFICATION**

(45) Date of publication of patent specification: **15.09.82**

(21) Application number: **78300184.5**

(22) Date of filing: **21.07.78**

(51) Int. Cl.³: **C 07 D 337/14,**
C 07 D 409/06,
C 07 D 417/06,
A 61 K 31/38

(54) **7-and 8-substituted -10,11-dihydro-11-oxodibenzo (b,f) thiepin derivatives, their production and their pharmaceutical compositions.**

(30) Priority: **26.07.77 US 819200**
**23.06.78 US 917211**

(43) Date of publication of application:
**07.03.79 Bulletin 79/5**

(45) Publication of the grant of the patent:
**15.09.82 Bulletin 82/37**

(84) Designated Contracting States:
**BE CH DE FR GB LU NL SE**

(56) References cited:
**NL - A - 74 11 917**
**NL - A - 77 13 373**

(73) Proprietor: **MERCK & CO. INC.**
**126, East Lincoln Avenue P.O. Box 2000**
**Rahway, New Jersey 07065 (US)**

(72) Inventor: **Rokach, Joshua**
**416 Canterbury Place**
**Chomedey-Laval Quebec (CA)**
Inventor: **Rooney, Clarence Stanley**
**2902 Hickory Hill Drive**
**Worcester Pennsylvania 19440 (US)**
Inventor: **Cragoe, Edward Jethro**
**2211 Oak Terrace Drive**
**Landsdale Pennsylvania 19446 (US)**

(74) Representative: **Crampton, Keith John Allen et al,**
**D YOUNG & CO 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 000 978 B1

7- and 8-substituted-10,11-dihydro-11-oxodibenzo[b,f]thiepin derivatives, their production and their pharmaceutical compositions

This invention relates to prostaglandin antagonists. Prostaglandin antagonists are useful in treating a variety of conditions, such as allergic asthma where excessive contractile activity of prostaglandins and prostaglandin biosynthetic intermediates occur. The prostaglandin antagonists of the present invention are 7- and 8-substituted-10,11-dihydro-11-oxodibenzo[b,f]thiepins having the structural formula:

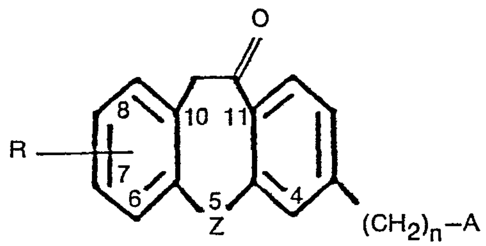

I

in which $n$ is 0 or an integer from 1 to 4; Z is thio, sulfinyl, or sulfonyl; R is in the 7-position or the 8-position 2 and is hydrogen, chlorine, bromine, fluorine, iodine, amino, $C_{1-4}$ alkyl, $C_{1-4}$ alkanoyl, hydroxyl, $C_{1-4}$ alkoxy, mercapto, $C_{1-4}$ alkylthio, $C_{1-4}$ alkylsulfinyl, $C_{1-4}$ alkylsulfonyl, trifluoromethyl, trifluoromethylthio, cyano, carboxy, nitro, $C_{1-4}$ alkylamino or di($C_{1-4}$ alkyl)amino; A is 5-tetrazolyl, 3-hydroxy-1,2,5-thiadiazol-4-yl, 4-hydroxy-2,5-dioxo-$\Delta^3$-pyrrolin-3-yl or

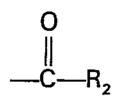

where $R_2$ is hydroxy, $C_{1-4}$ alkoxy, N,N-di($C_{1-4}$ alkyl)amino-($C_{1-4}$ alkoxy), $C_{1-4}$ hydroxyalkoxy, carboxy-($C_{1-4}$ alkoxy), amino, $C_{1-4}$ alkylamino, di($C_{1-4}$ alkyl)amino, $C_{1-4}$ alkylsulfonylamino, carboxy($C_{1-4}$ alkyl)-amino, carbamoyl($C_{1-4}$alkyl)amino or 2-imino-3-methylthiazolidine with the proviso that $R_2$ is not hydroxy when $n$ is 0, R is hydrogen and Z is thio; and the pharmaceutically acceptable salts thereof.

It should be noted that the compound 10,11-dihydro-11-oxo-dibenzo[b,f]thiepin-3-carboxylic acid is excluded from this invention because it is disclosed, under the name 10,11-dihydrodibenzo[b,f]thiepin-10-one-7-carboxylic acid, in the article by Pelz et al on pp 3936 to 3943 of Collection Czechoslov. Chem. Commun. Vol 34 (1969) (see page 3941).

As used herein, the term halogen (or halo) includes chlorine, bromine, iodine and fluorine. Unless otherwise specifically stated, the terms loweralkyl and lower alkoxy include straight and branched chain alkyl and alkoxy groups having 1 to 4 carbon atoms in the alkyl or alkoxy moiety such as, for example, methyl, ethyl, $n$-propyl, isopropyl, $n$-butyl, methoxy, ethoxy, $n$-propoxy and isobutoxy. The term loweralkanoyl includes straight or branched chain alkanoyl groups having 1 to 4 carbon atoms in the alkanoyl moiety such as, for example, formyl, acetyl, propanoyl, butyryl and isobutyryl.

These dibenzo[b,f]thiepin derivatives antagonize the actions of contractile prostaglandins, such as $PGF_2$, $PGG_2$ and $PGH_2$ and $TXA_2$. The use of agents which act as prostaglandin antagonists offers new approaches to therapy in a number of disease states. For example, certain prostaglandins, such as $PGF_{2a}$, $PGG_2$ and $PGH_2$, are potent contractants of bronchial muscle. Indeed human asthmatics have been shown to be especially sensitive to the bronchial constricting action of $PGF_{2a}$.

In addition to the involvement of contractile prostaglandins in chronic obstructive lung disease (or asthma), prostaglandins are known to play a role in other allergic conditions, as well as inflammation, diarrhea, hypertension, angina, platelet aggregation, cerebral spasm, premature abortion, and dismenorrhea.

In addition to the prostaglandin antagonist actions, the dibenzo[b,f]thiepins of this invention are antagonists of slow reacting substance of anaphylaxis (SRS—A). This contractile substance is released in the lung tissue in allergic asthma, and antagonism of its actions contributes to alleviation of this disease.

The dibenzo[b,f]thiepins of this invention are prepared according to the following general reaction scheme.

An appropriately substituted mercaptobenzoic acid II is reacted with m-dibromobenzene III ($R_3$=Br) to obtain the 0-(3-bromophenyl)benzoic acid IV. Or alternatively, an appropriately substituted o-bromobenzoic acid II ($R_2$=Br) is reacted with m-bromobenzenethiol ($R_3$=SH) to give IV,

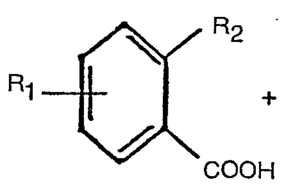 + 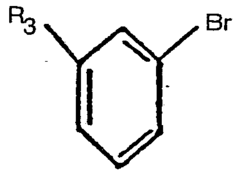 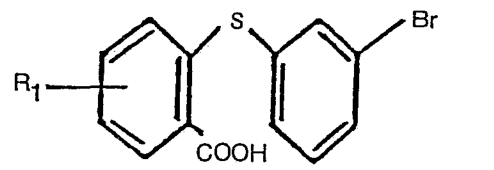

II III IV

where $R_1$ is hydrogen, nitro, amino, $C_{1-4}$ alkanoyl, hydroxyl, $C_{1-4}$ alkoxy, $C_{1-4}$ alkylsulfinyl, $C_{1-4}$ alkylsulfonyl, $C_{1-4}$ alkyl, trifluoromethyl or trifluoromethylthio, $R_2$ and $R_3$ are different: one of them is mercapto and the other is bromine.

Generally, the sulfide-forming reaction is carried out according to the methods described by Jilek et al., Monatsh. Chem. 96, 200 (1965), Protiva et al, Czech. Patent 121,337 C.A. 68 (105, 247t, 1968) and U.S.P. 3,711,489, and by other procedures well known in the art.

The resulting o-(3-bromophenylthio)benzoic acid (IV) is reduced to the alcohol, brominated, and the bromo replaced with cyano. The cyano derivative is then hydrolyzed to the carboxylic acid V.

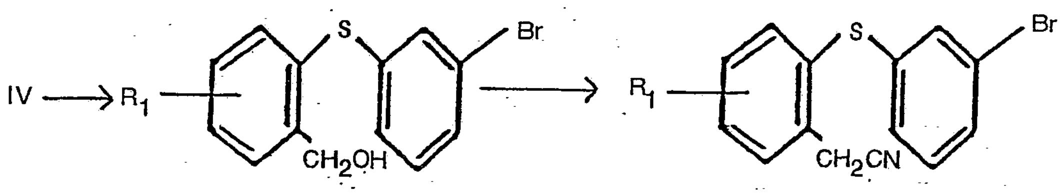

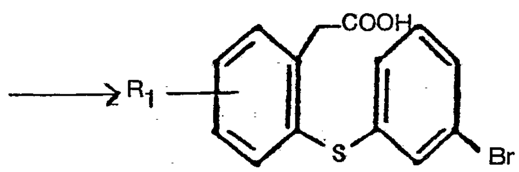

V

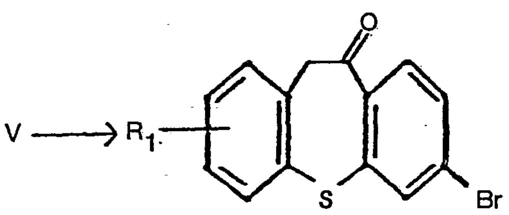

VI

The carboxylic acid V is transformed into the 3-bromo-11-oxo-10,11-dihydrodibenzo[b,f]thiepin by first conversion to the acid halide with thionyl or phosphoryl halide followed by Friedel-Crafts cyclization with a Lewis acid such as aluminum chloride to give VI. Reduction of the ketone VI with alkali metal borohydrides, followed by heating with catalytic amounts of a mineral acid, such as sulfuric acid or toluenesulfonic acid provides the 3-bromodibenzo[b,f]thiepin VII.

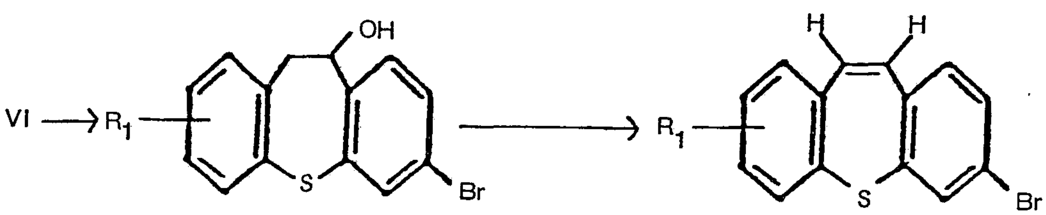

VII

The 3-bromo derivative VII is then converted to the 3-nitrile VIII by reaction with cuprous cyanide in a high boiling polar solvent such as dimethylformamide, N-methylpyrrolidone and the like.

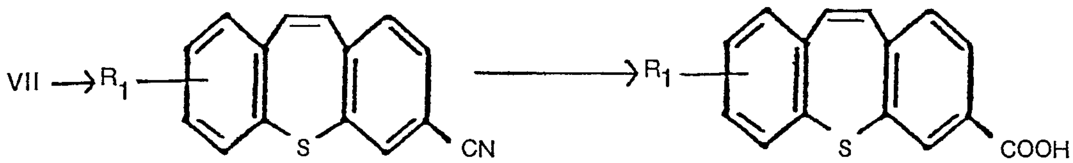

VIII IX

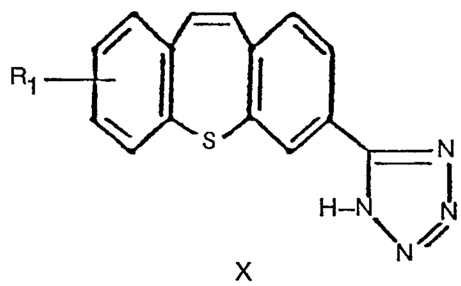

X

The 3-cyano derivative VIII may be hydrolyzed with aqueous mineral acid or base to give the dibenzo[b,f]thiepin-3-carboxylic acid IX. The 3-cyano compound VIII may also be reacted with azide ion at reflux in an inert solvent such as dimethylformamide, hexamethylphosphorictriamide and the like for 1/4 to 18 hours to give the tetrazole derivative X. Alternatively, the cyano intermediate VIII may be oxidized with organic peroxides such as peroxy acids, for example, m-chloroperbenzoic acid and the like, in a stepwise fashion to the corresponding sulfoxide XI and sulfone XII, controlling the molar ratio of oxidant to reductant. This determines the oxidation level of the sulfur. For example, a 1:1 molar ratio results largely in the production of sulfoxide XI. In contrast, a 2 to 3 molar excess of oxidant results in a yield predominantly comprising the sulfone XII.

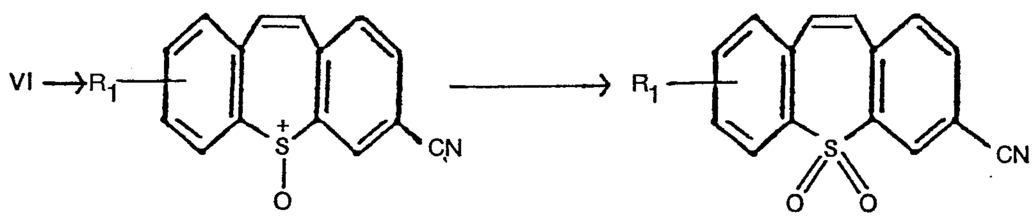

XI XII

Hydrolysis of XI and XII using aqueous mineral acid or alkali provides the corresponding carboxylic acids XIII and XIV.

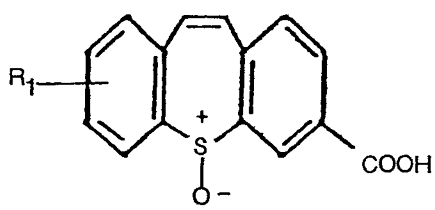

XIII

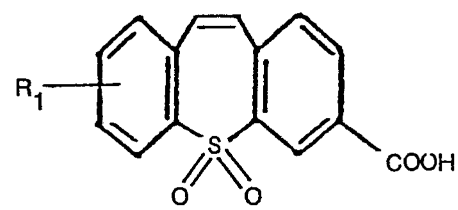

XIV

Reaction of XI and XII with azide ion as described above provides the tetrazoles XV and XVI, respectively.

XI 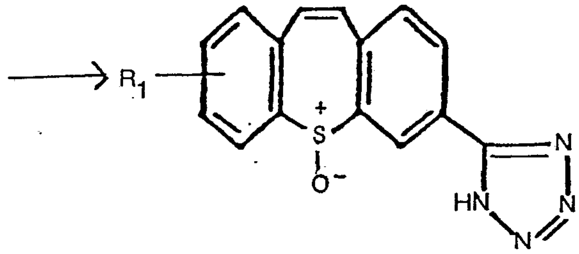

XV

XII 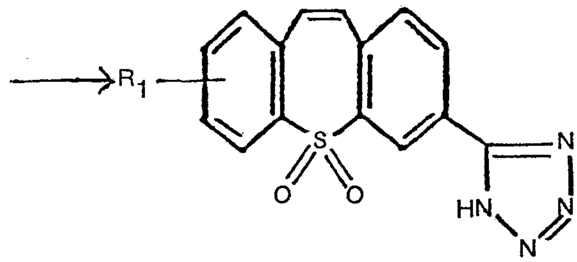

XVI

Compounds of type I where the 10—11 double bond is saturated are prepared from intermediate VI in which the 3-bromo is converted to a cyano derivative XVII, then hydrolyzed with mineral acid to the acid XVIII and esterified to the ester XIX, where R′ is loweralkyl.

VI 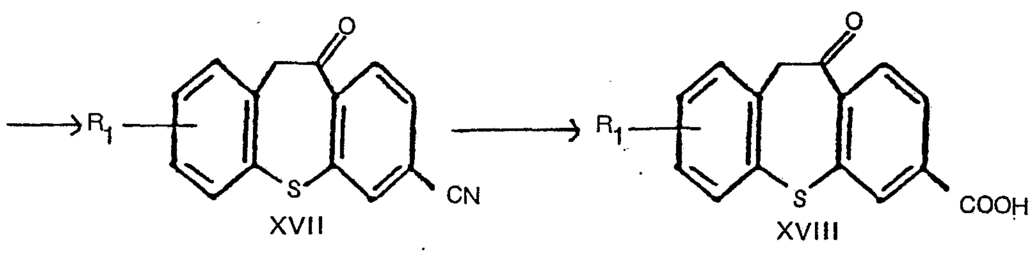

XVII XVIII

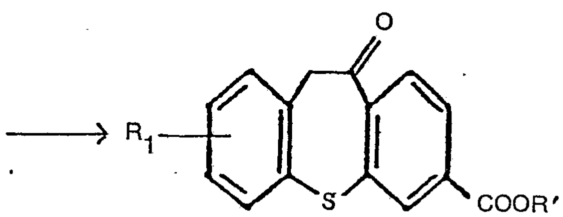

XIX

The ester XIX is reduced by conventional methods, e.g. Wolff-Kishner, to compound XXII or better by reduction with $NaBH_4$ to XX followed by $PBr_3$ reaction to XXI, then reduction with $NaBH_4$ in solvent such as sulfolane to XXII.

XIX 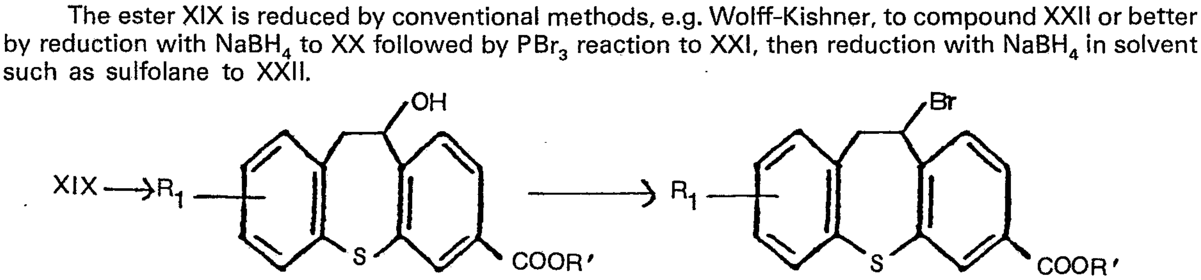

XX XXI

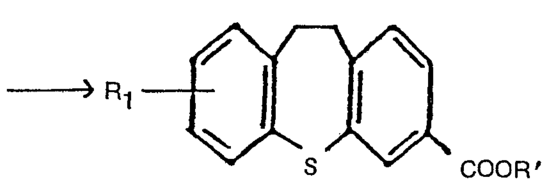

XXII

Compound XXII may be hydrolyzed with aqueous mineral acid or base to give the 10,11-dihydro[b,f]thiepin-3-carboxylic acid XXIII. Compound XXII may also be oxidized with one equivalent of organic peroxides, such as peroxy acids, for example, m-chloroperbenzoic acid and the like, to yield the sulfoxide XXIV, which can then be hydrolyzed with mineral acid or base to provide the carboxylic acid XXV.

XXII 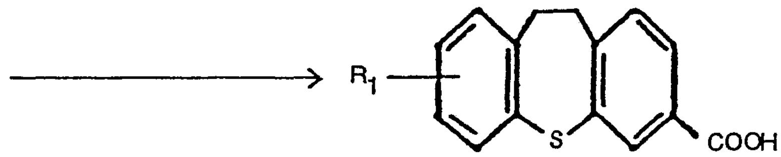

XXIII

XXII 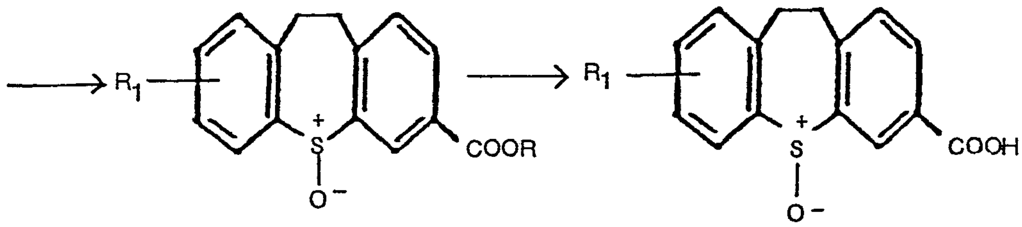

XXIV XXV

The acid XXIII may be oxidized with excess organic peroxides such as hydrogen peroxide in acidic solvents such as acetic acid at temperatures ranging from 0°—100°C. to yield compound XXVI.

XXIII 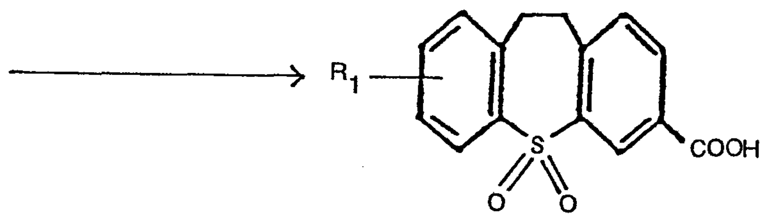

XXVI

Compound VI may be reacted with phosphorus tribromide followed by reduction with $NaBH_4$ in solvents, such as sulfolane, and reaction with cuprous cyanide in high boiling polar solvents, such as dimethylformamide, N-methylpyrrolidone and the like, to yield XXVII.

VI 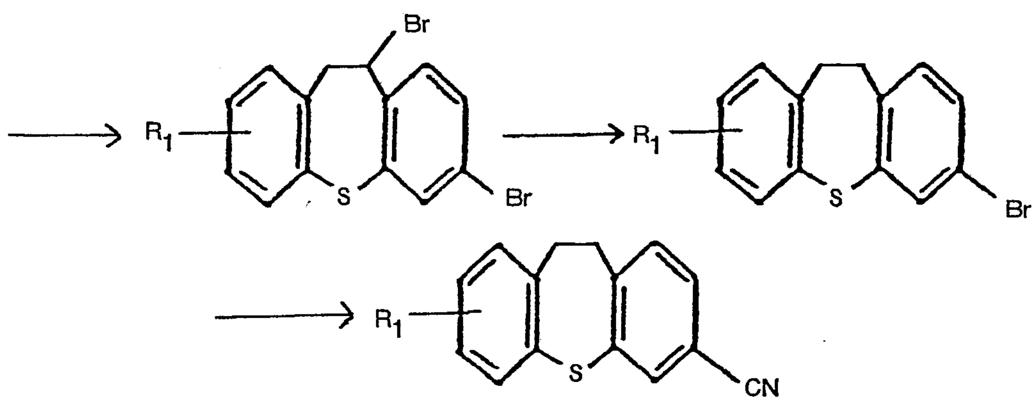

XXVII

The 3-cyano derivative XXVII may be reacted with azide ion at reflux in an inert solvent such as dimethylformamide or in THF with the addition of Lewis acid, such as $AlCl_3$, to yield the tetrazole

derivative XXVIII. Compound XXVII can also be oxidized with excess peroxy acid, such as m-chloroperbenzoic acid followed by the tetrazole forming reaction to yield XXIX.

XXVII
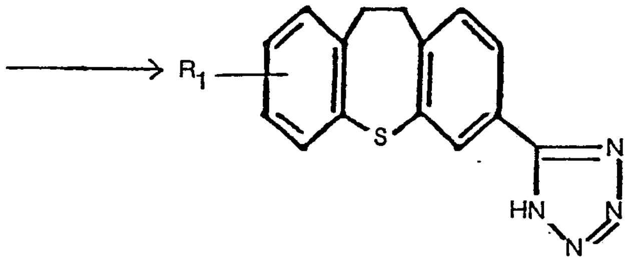
XXVIII

XXVII
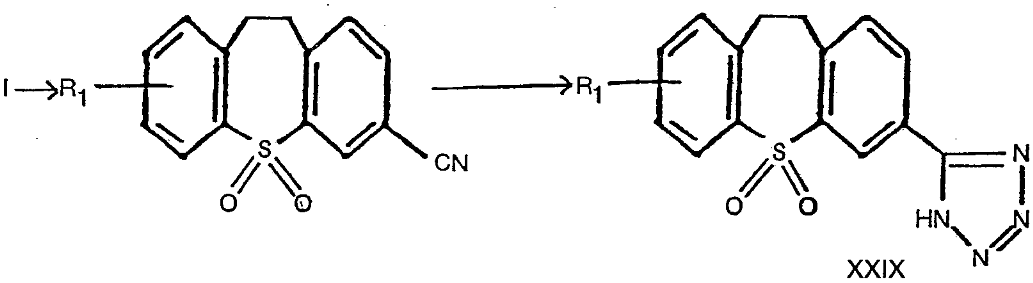
XXIX

Tetrazole XXVIII may be oxidized with peroxides such as hydrogen peroxide in acidic medium, such as acetic acid, to yield compound XXX.

XXVIII
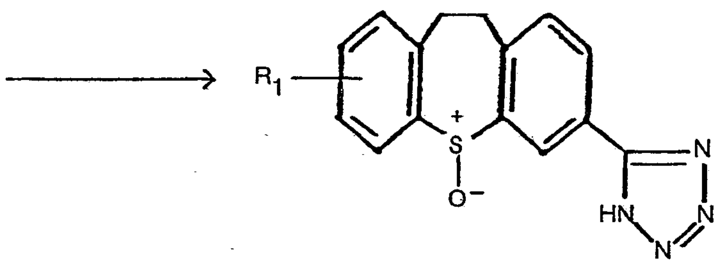
XXX

XVII may also be oxidized with one equivalent of organic peroxide, such as peroxy acids, for example, m-chloroperbenzoic acid followed by mineral acid or base hydrolysis to yield XXXI.

XVII
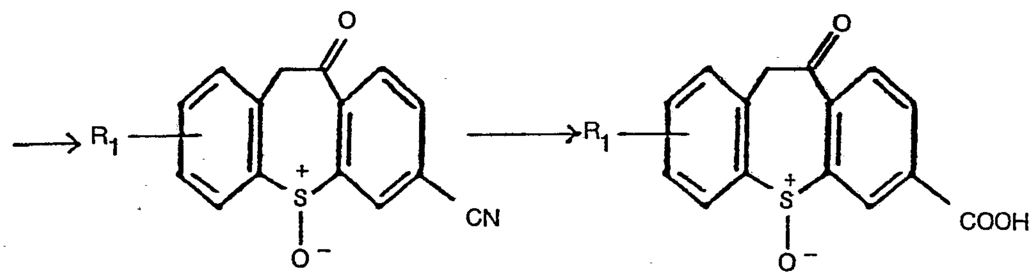
XXXI

XVII can also be reacted with azide ion at reflux in an inert solvent such as dimethylformamide or in THF with the addition of a Lewis acid such as $AlCl_3$ to yield the tetrazole XXXII.

XVII
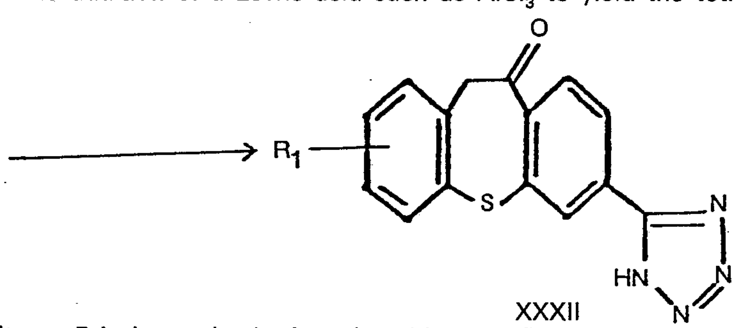
XXXII

Substituent R in I can also be introduced by modification of the nitro group in VIII ($R=NO_2$) and XXVII ($R=NO_2$) by known procedures. For example, XXXIII can be reduced with stannous chloride in

acidic medium, hydrochloric acid and the like, to yield XXXIV which can be hydrolyzed with mineral acids or bases to XXXV.

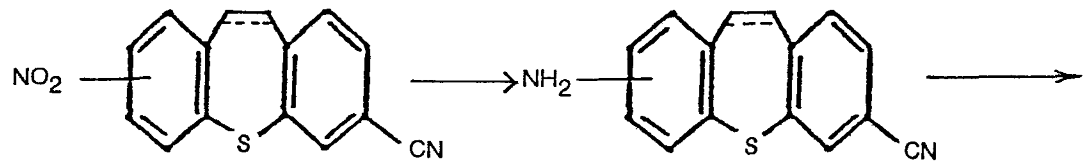

XXXIII XXXIV

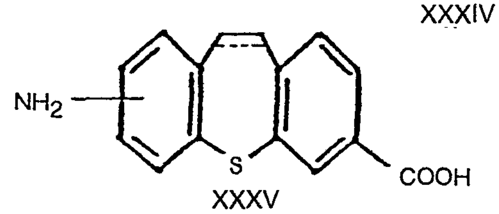

XXXV

Alternatively, XXXIII may be oxidized with peroxides, for example, m-chloroperbenzoic acid to yield XXXVI which can be reduced to XXXVII and then hydrolyzed with mineral acids or bases to XXXVIII.

XXXIII

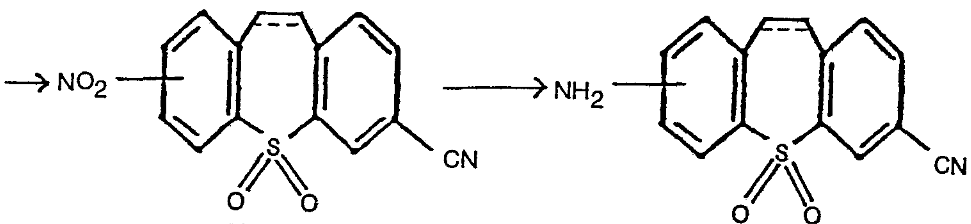

XXXVI XXXVII

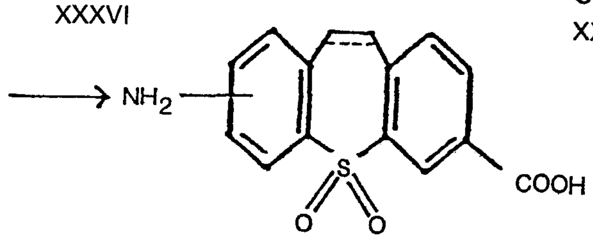

XXXVIII

Intermediate XXXIV can be reacted with sodium nitrite in mineral acid to the diazonium salt XXXIX, where X is a mineral acid counter ion, for example, $Cl^-$, $HSO_4^-$, $BF_4^-$ and the like, which on reaction with CuCl and $CuCl_2$ yields intermediate XL which can be hydrolyzed to the acid XLI. Intermediate XL may also be oxidized to the sulfone derivative, then followed by a hydrolysis to the carboxylic acid XLII.

XXXIV

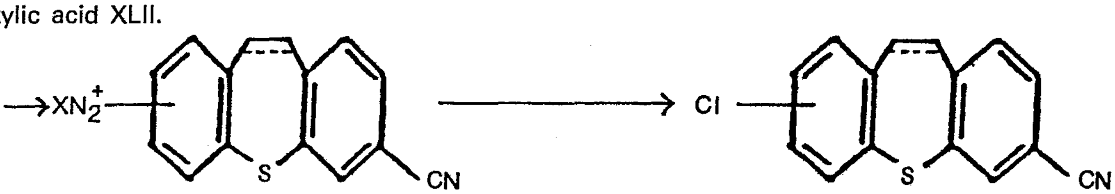

XXXIX XL

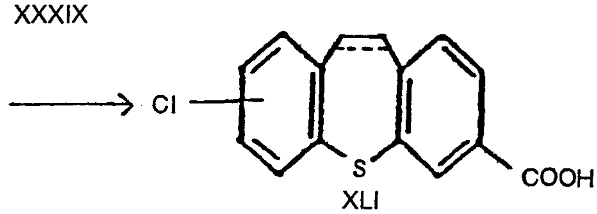

XLI

XL

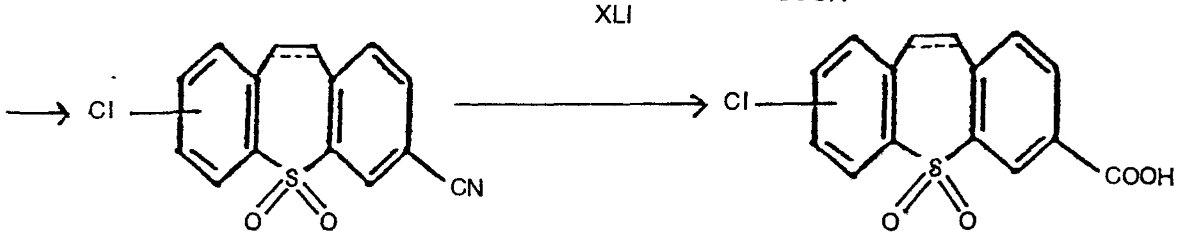

XLII

Derivative XXXIX can be hydrolyzed with a solution of sulfuric acid 10 to 50% in strength at temperatures ranging from 0°—90°C. to yield XLIII. XXXIX may also be reacted with potassium thioxanthate at temperatures from 40°—70°C. followed by basic hydrolysis to yield the thiol acid XLIV.

XXXIX

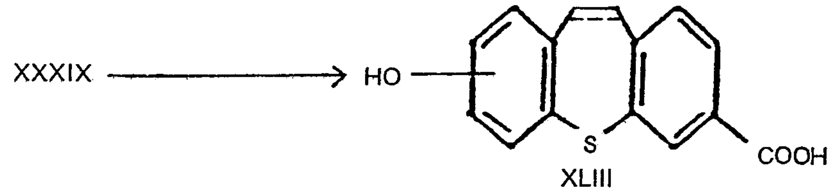

XLIII

XXXIX

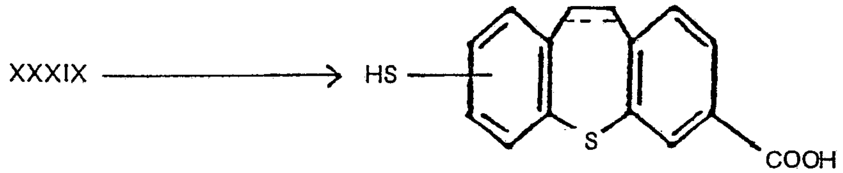

XLIV

Compound XXXVII can be transformed in the usual manner to the diazonium salt XLV. XLV can be reacted as described above to yield compounds XLVI and XLVII.

XXXVII

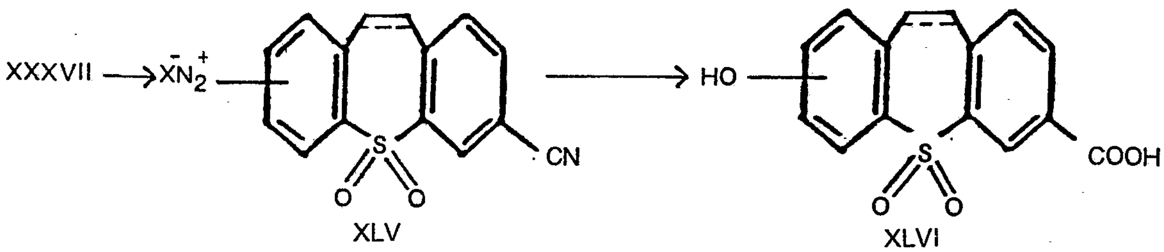

XLV XLVI

XLV

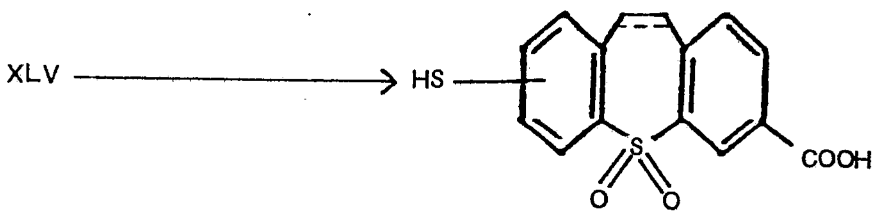

XLVII

Compounds XLIII, XLIV, XLVI, XLVII can be reacted with alkyl halides RX in which R is a lower alkyl $C_1$ to $C_4$, benzyl, and X is a leaving group such as Cl, Br, I,

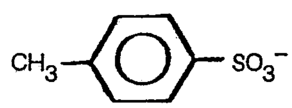

in the presence of bases such as alkali carbonate, hydroxides, and the like, in solvents such as dimethylformamide, at temperatures ranging from 30°—160°C. to yield XLVIII, XLIX, L and LI, respectively.

XLIII 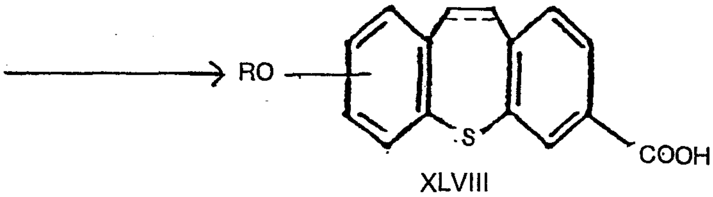

XLVIII

XLIV 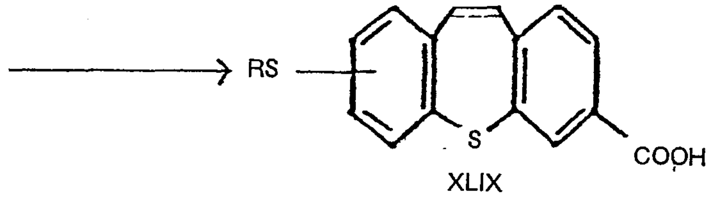

XLIX

XLVI 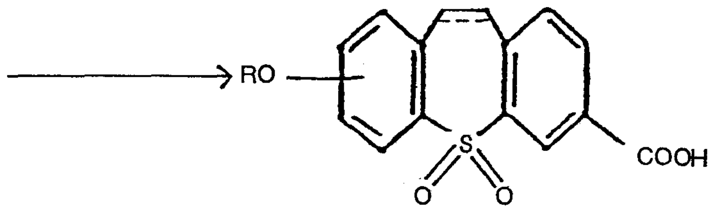

L

XLVII 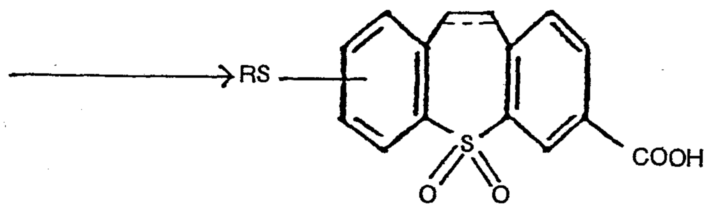

LI

Compound XLIX can in a controlled oxidation with peroxides such as hydrogen peroxide or organic peroxy acids such as m-chloroperbenzoic acid, yield compound LII. LI may be oxidized with one equivalent of organic peroxides such as m-chloroperbenzoic acid or with hydrogen peroxide in hydroxylic solvents such as alcohols, organic acids such as acetic acid, at temperatures below 30°C., to yield LIII. Compounds XLIX, LI and LIII may also be oxidized with excess organic peroxides such as m-chloroperbenzoic acid at room temperature, or with peroxides such as hydrogen peroxide in acidic medium such as acetic acid at temperatures between 80° and 100°C. to yield LIV.

XLIX 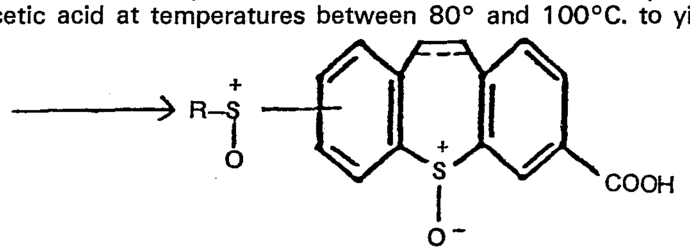

LII

LI 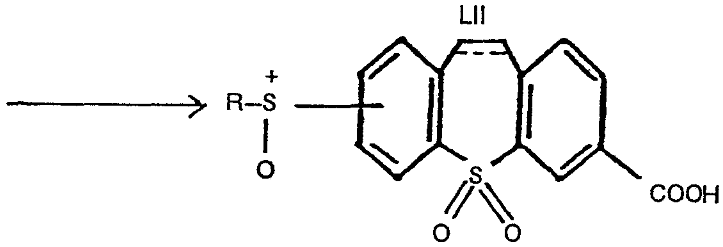

LIII

XLIX, LI, LIII 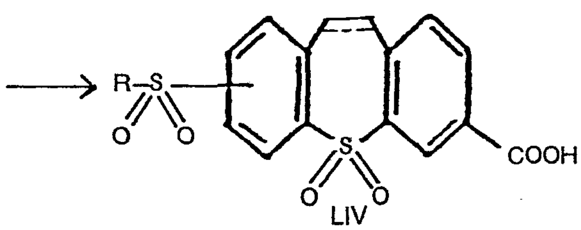

LIV

Specific introduction of substituents in position 8 in I can also be achieved. For example, XXVII ($R_1$=H) can react with alkanoyl halide RCOX or alkanoic anhydride RCOOCOR in which R is a lower alkyl $C_1$ to $C_4$ and X is chloro or bromo, to yield the substituted acyl LV which upon acid or base hydrolysis affords acid LVI.

XXVII ($R_1$ = H) 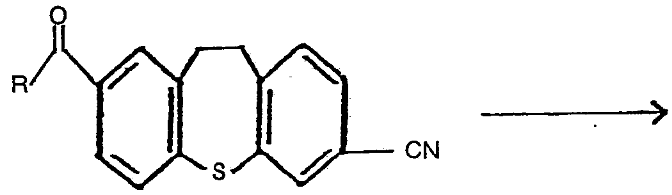

LV

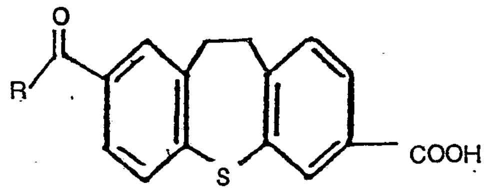

LVI

LV can be oxidized with oxidizing agents such as in-chloro perbenzoic acid stepwise to yield sulfoxide LVII and sulfone LVIII which are hydrolyzed under acidic or basic conditions to afford acids LIX and LX respectively.

LV 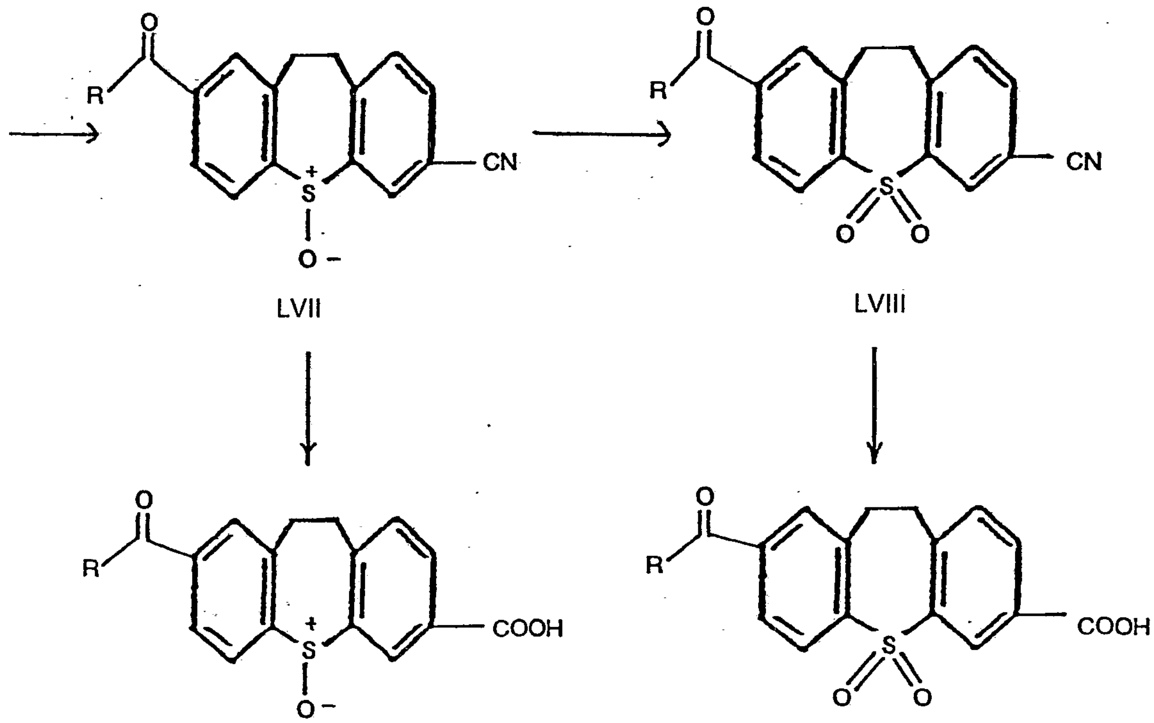

LVII LVIII

LIX LX

Compound LVI can be reacted with hydroxylamine hydro chloride with presence of base to yield oxime LXI which on a Beckman rearrangement, yields the acylamino compound LXII which upon hydrolysis yields amino acid LXIII.

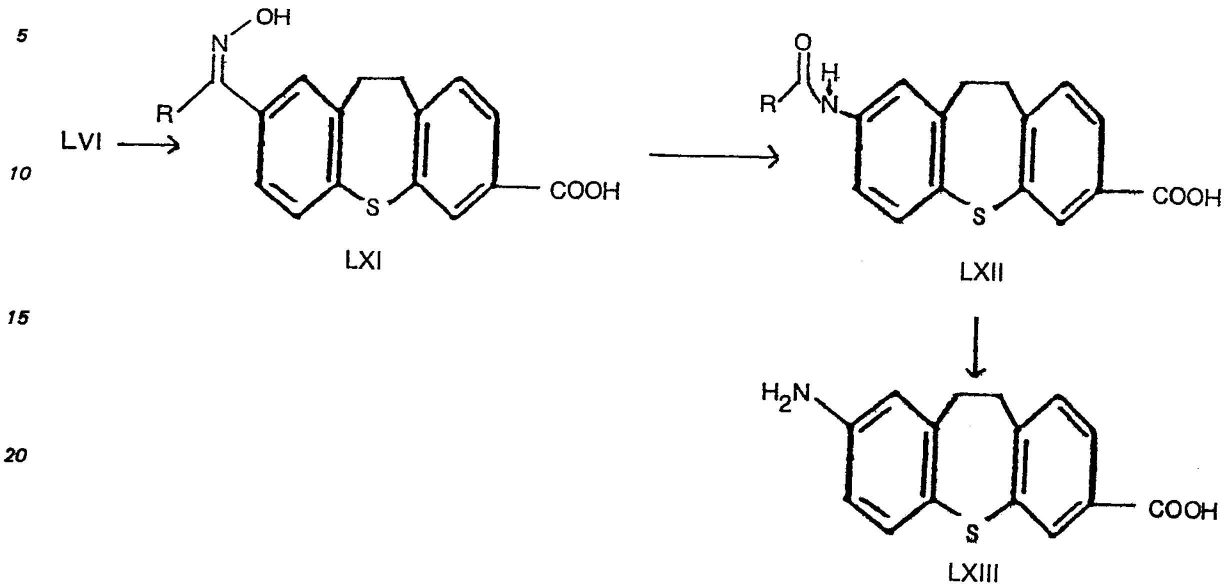
LVI

LXI

LXII

LXIII

Compound LXII can be oxidized with hydrogen peroxide in acetic acid stepwise to yield the corresponding sulfoxide LXIV and sulfone LXV which upon hydrolysis afford the acids LXVI and LXVII.

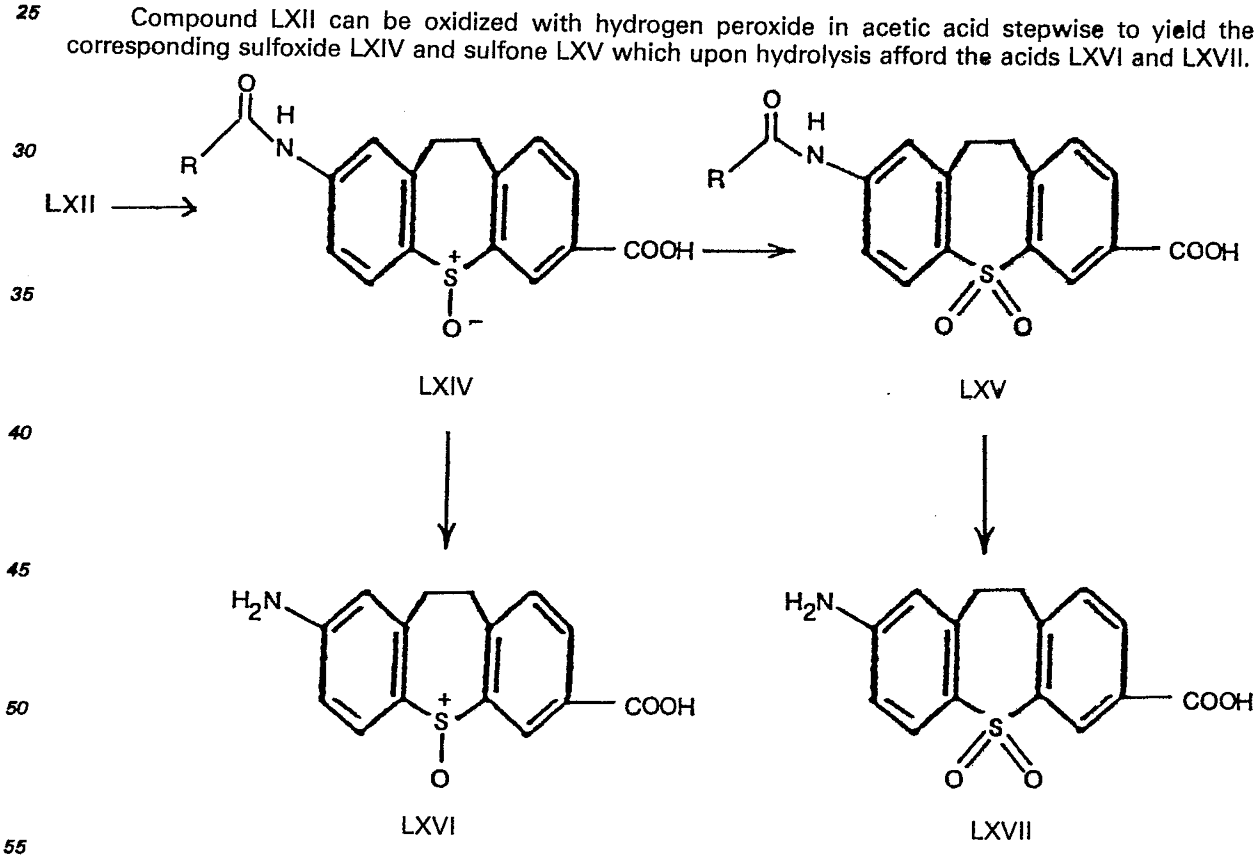
LXII

LXIV

LXV

LXVI

LXVII

Compounds LXIII, LXVI, LXVII can be treated in various Sandmeyer reactions as described earlier to yield I substituted in the 8 position.

Compound LVI ($R{=}CH_3$) when treated with sodium hypochlorite and base at temperatures from 0°—70° for half an hour yield the diacid LXVIII. When the process is carried for 2 days under the same conditions LXIX is obtained.

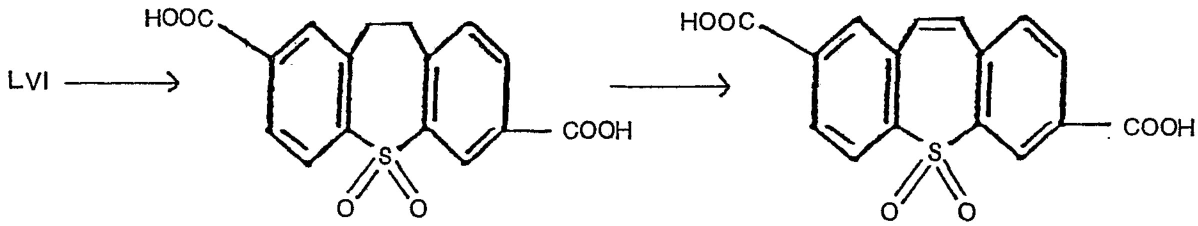


LXVIII LXIX

Compounds LVI, LIX, LX can be reduced with sodium borohydrid to afford the corresponding alcohols, LXX, LXXI, LXXII.

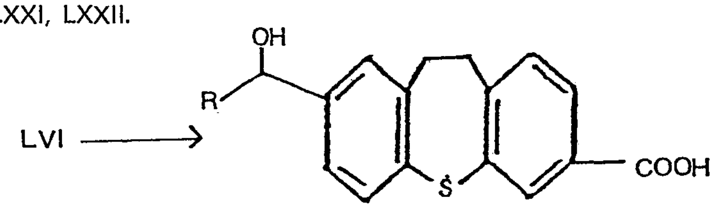


LXX

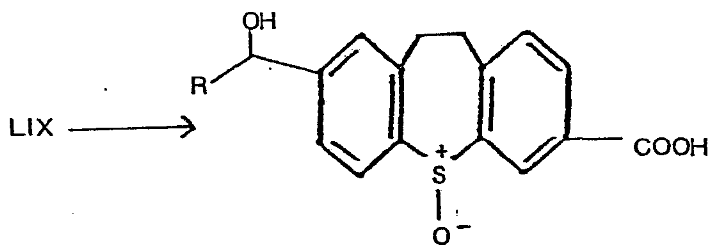


LXXI

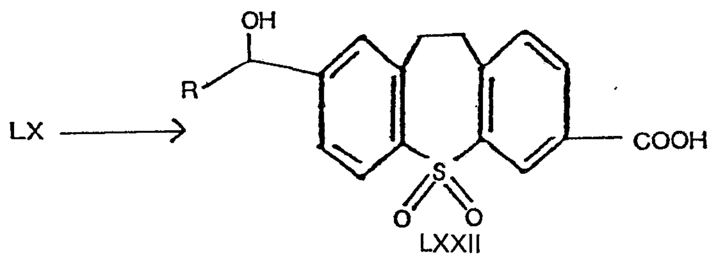


LXXII

It will be obvious to those skilled in the art that the nitrile, XVII, can be substituted for the nitrile starting material, XXVII, in the foregoing reaction sequences in order to prepare correspondingly substituted 10,11-dihydro-11-oxodibenzo[b,f]thiepins.

In addition to their therapeutic properties as noted above, the 3-carboxylic acid derivatives of this invention serve as valuable intermediates in the preparation of other variously substituted thiepins of formula I. Thus, for example, the 3-carboxylic acid of formula XVIII ($R_1$=R as defined in formula I) may be converted readily into the corresponding acid halide, preferably the acid chloride, by treating the carboxylic acid with a thionyl halide, preferably thionyl chloride. The resulting 3-halocarbonyl 10,11-dihydro-11-oxodibenz[b,f][1,4]thiepin i.e., the 3-chlorocarbonyl compound of formula LXXIII then may be treated with various well-known reagents to form desired ester and amide derivatives. These reactions are illustrated in the following reaction scheme wherein R is as previously defined, it being understood that they are equally applicable to the 3-carboxylic acids of formula IX or XXIII.

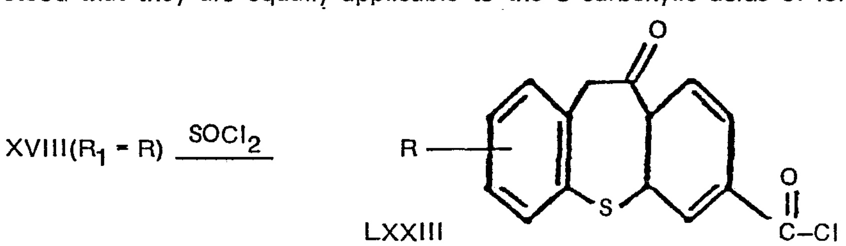


LXXIII

Thus, for example, the chlorocarbonyl compound of formula LXXIII may be treated:

(a) with a loweralkanol such as, for example, methanol, ethanol, 2-propanol, butanol and 2-butanol, to form the corresponding loweralkyl ester, LXXIV:

LXXIII

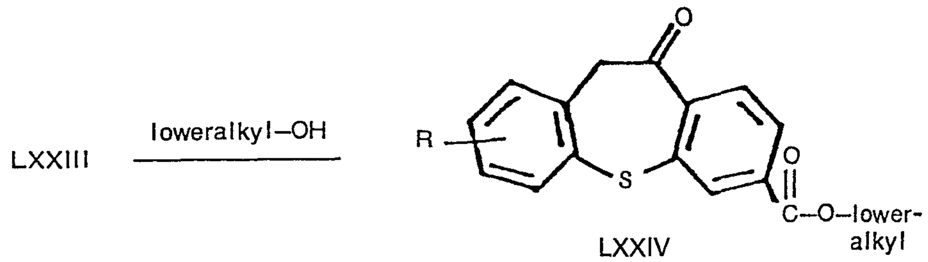


LXXIV

(b) with ammonia to form the corresponding carboxamide, LXXV:

LXXIII

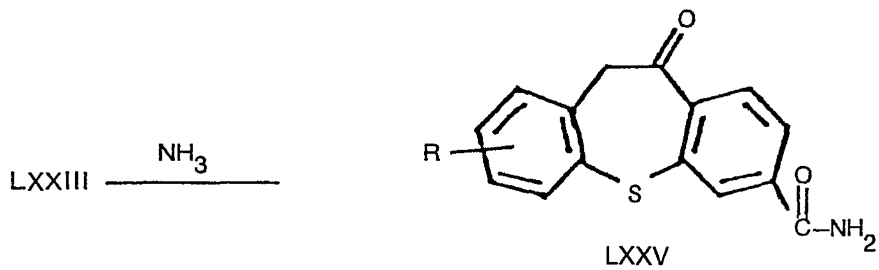


LXXV

(c) with an N-loweralkylamine such as for example, methylamine, ethylamine, propylamine, isopropylamine and butylamine, or an N,N-diloweralkylamine such as, for example, dimethylamine, diethylamine, dipropylamine and dibutylamine, to form the corresponding N-loweralkylcarboxamide LXXVI or N,N-diloweralkylcarboxamide, LXXVII:

LXXIII

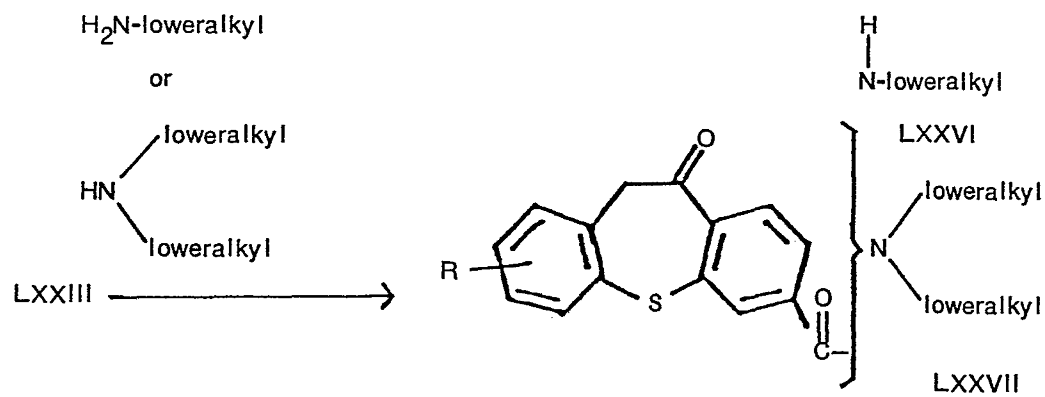


LXXVI

LXXVII

(d) with a loweralkylsulphonamide such as, for example, methane sulphonamide, ethane sulphonamide, propane sulphonamide and butane sulphonamide, to form the corresponding N-loweralkylsulfonylcarboxamide, LXXVIII:

LXXIII

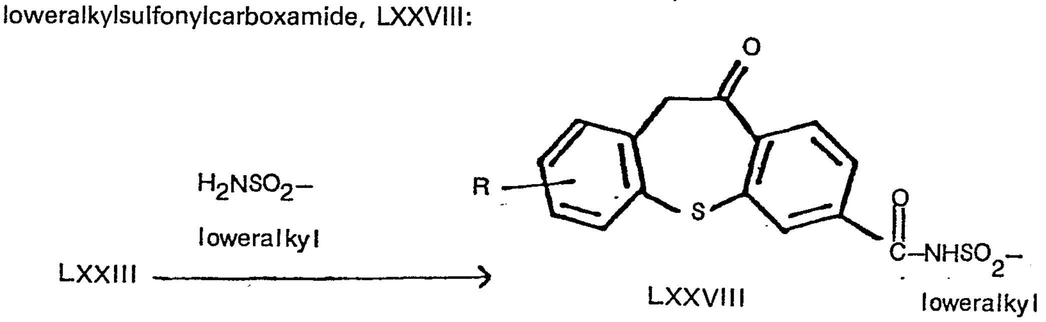


LXXVIII

(e) with 2-imino-3-methylthiazolidine to form the corresponding (3-methyl-2-thiazolidinylidene)carboxamide, LXXIX:

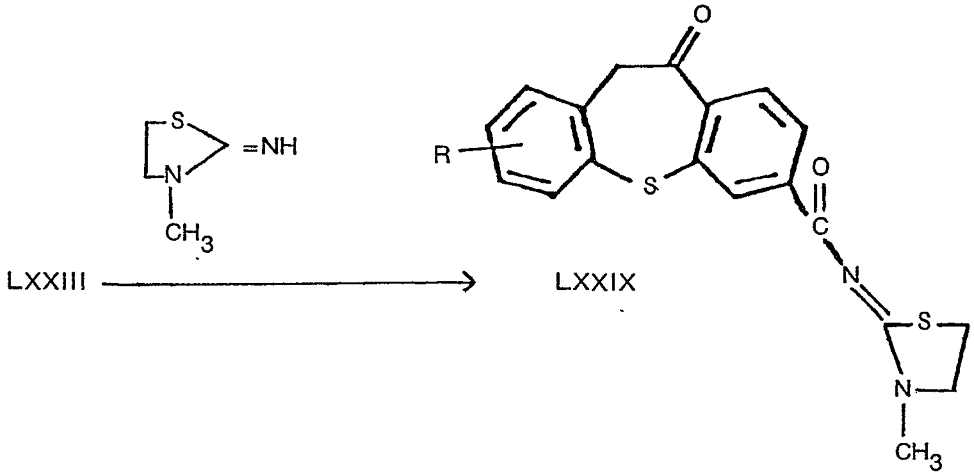

LXXIII

LXXIX

(f) with a loweralkyldiol such as, for example ethylene glycol, trimethylene glycol and 1,4-butanediol, to form the corresponding hydroxyloweralkylester, LXXX:

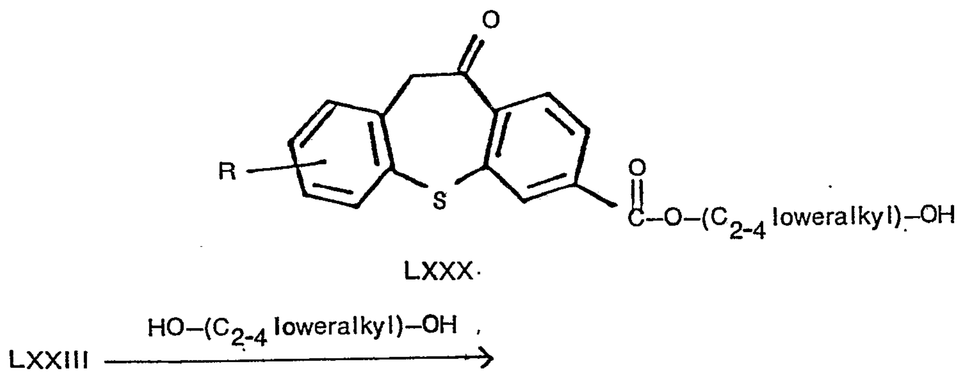


LXXX

LXXIII

(g) with an N,N-diloweralkylaminoloweralkanol such as, for example, N,N-dimethylethanolamine, N,N-diethylethanolamine, 3-N,N-dimethylaminopropan-1-ol and 4-N,N-diethylaminobutan-1-ol, to form the corresponding N,N-diloweralkylaminoloweralkyl ester, LXXXI:

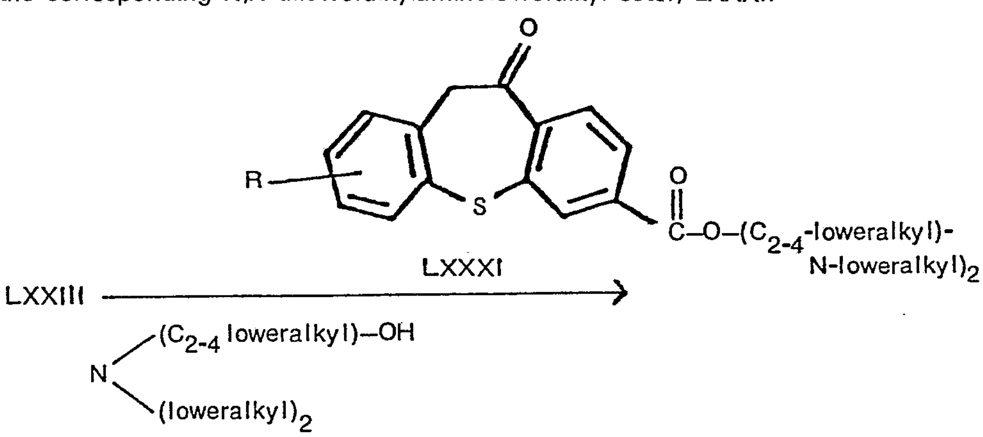

LXXXI

LXXIII

(h) with an amino acid such as, for example, glycine, alanine and valine, to form the corresponding N-carboxyloweralkylcarboxamide, LXXXII:

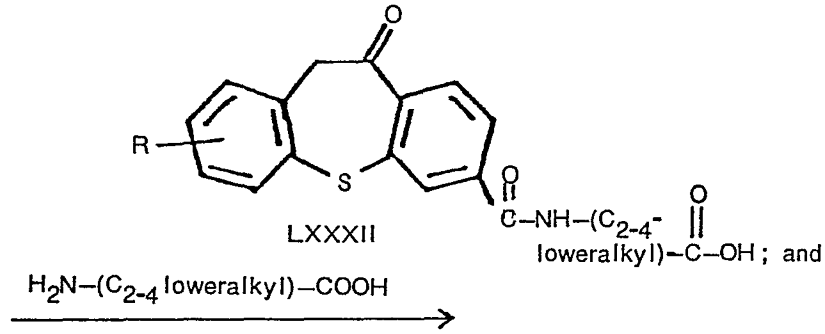


LXXXII ; and

LXXIII

(i) with an alkali metal salt of a hydroxyloweralkanoic acid such as, for example, hydroxyacetic acid, 3-hydroxybutyric acid and $\beta$-hydroxypropionic acid, to form the corresponding carboxyloweralkyl ester, LXXXIII:

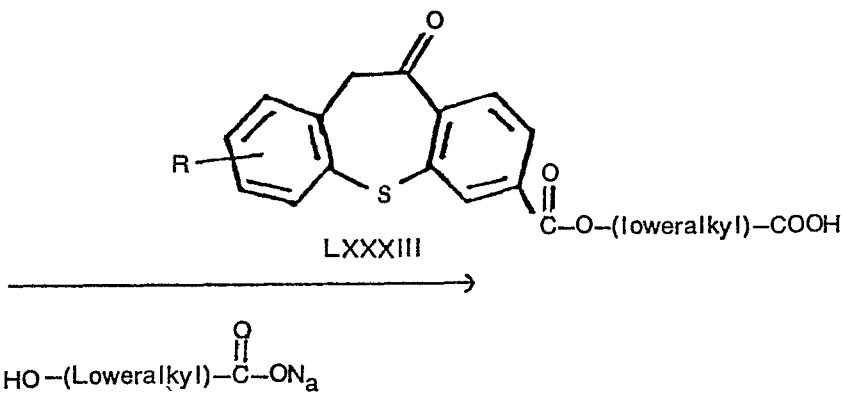


LXXXIII

LXXIII

Where the corresponding sulfinyl or sulfonyl derivatives are desired, the corresponding 11-oxide or 11,11-dioxide 3-carboxylic acid may be substituted for starting material XVIII in the foregoing reaction sequence. Alternatively, it will be clear to those skilled in the art that the product esters and amides obtained in the foregoing reaction sequence may be oxidized by the techniques already described to obtain the corresponding sulfinyl or sulfonyl derivatives.

Those thiepins of this invention wherein the substituent at the 3-position is 3-hydroxy-1,2,5-thiadiazol-4-yl are prepared by refluxing a 3-cyano intermediate (a compound of formula XVII where $R_1$=R as defined in formula I, for example) in formic acid in the presence of Raney nickel alloy for 1 to 2 hours in order to obtain the corresponding 10,11-dihydro-11-oxodibenzo-[b,f]-thiepin-3-carboxaldehyde. The aldehyde product then is converted into the corresponding 3-(2-aminoacetonitrile) by treatment with sodium cyanide in an alcoholic solvent saturated with ammonia and in the presence of ammonium chloride and ammonium hydroxide. The reaction usually is conducted at room temperature and requires from 8 to 16 hours for completion. The aminoacetonitrile so produced is treated with concentrated hydrochloric acid at room temperature for 20 to 45 minutes in order to obtain the corresponding 3-(2-aminoacetamide) which then is treated with sulfur monochloride in dimethylformamide to obtain the desired 10,11-dihydro-11-oxo-dibenzo[b,f]-thiepin-3-(3-hydroxy-1,2,5-thiadiazol-4-yl) of formula LXXXIV. This reaction sequence is illustrated in the following diagram.

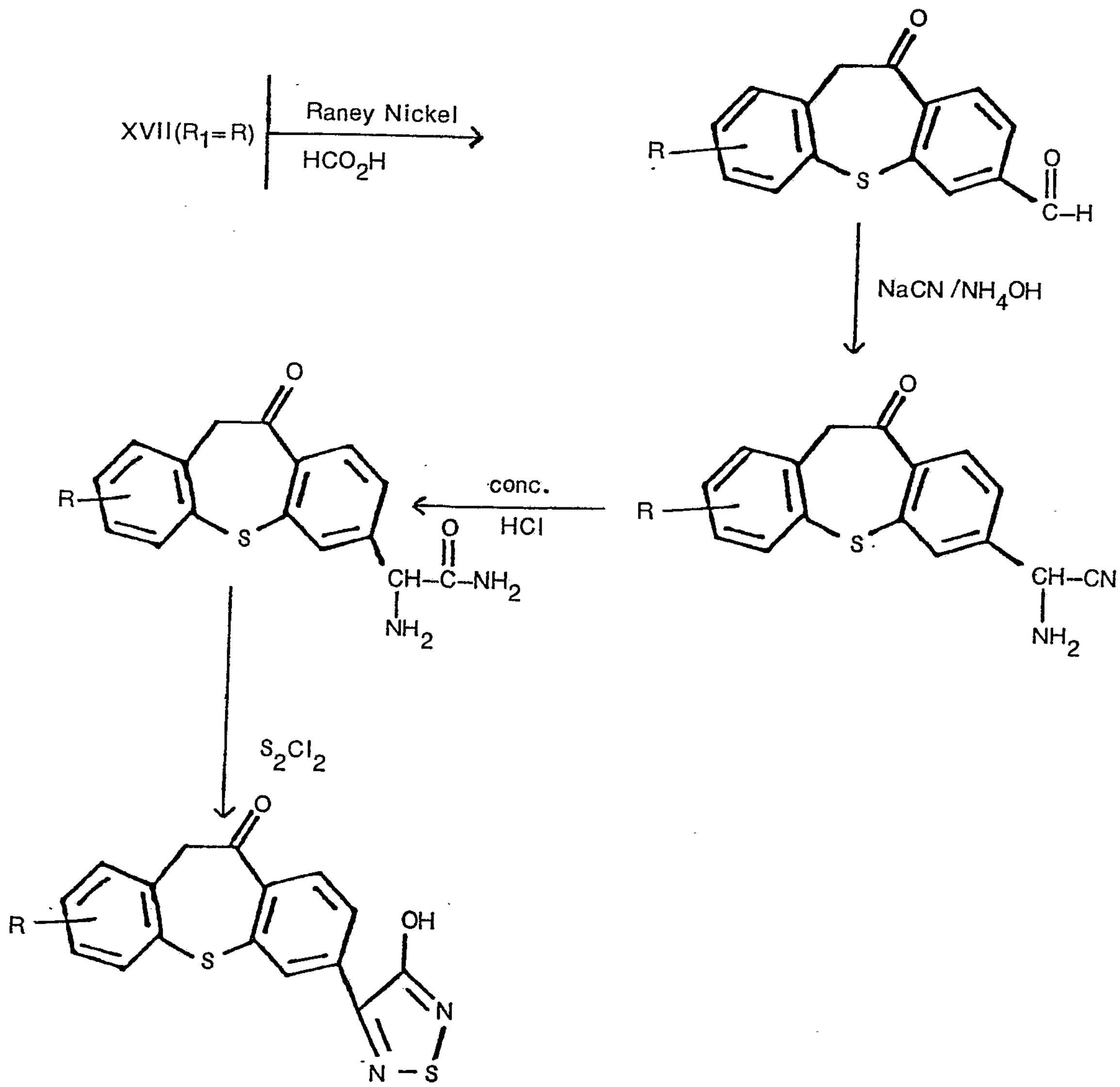


LXXXIV

The novel thiepins of this invention wherein the substituent at the 3-position is 4-hydroxy-$\Delta^3$-pyrroline-3-yl-2,5-dione are prepared from the appropriately substituted 3-carboxylic acid by reducing the acid to the corresponding alcohol with borane in tetrahydrofuran. The reaction conveniently is carried out at room temperature under an inert atmosphere and usually requires 2 to 4 hours for completion. The alcohol then is brominated with phosphorus tribromide and the bromomethyl compound so produced is treated with sodium cyanide to form the correspondiong 3-cyanomethyl derivative. These reactions may be carried out at room temperature and usually require from 1 to 3 hours for completion. The cyanomethyl intermediate then is hydrolyzed to the corresponding acetic acid which is treated with thionyl chloride followed by ammonia to form the corresponding 3-acetamide derivative by techniques already described. The acetamide then is treated with diethyloxalate in dimethylformamide in the presence of potassium *t*-butoxide to form the desired dibenzo[b,f]thiepin-3-(4-hydroxy-$\Delta^3$-pyrrolin-3-yl-2,5-dione), LXXXV. This reaction sequence is illustrated in the diagram following.

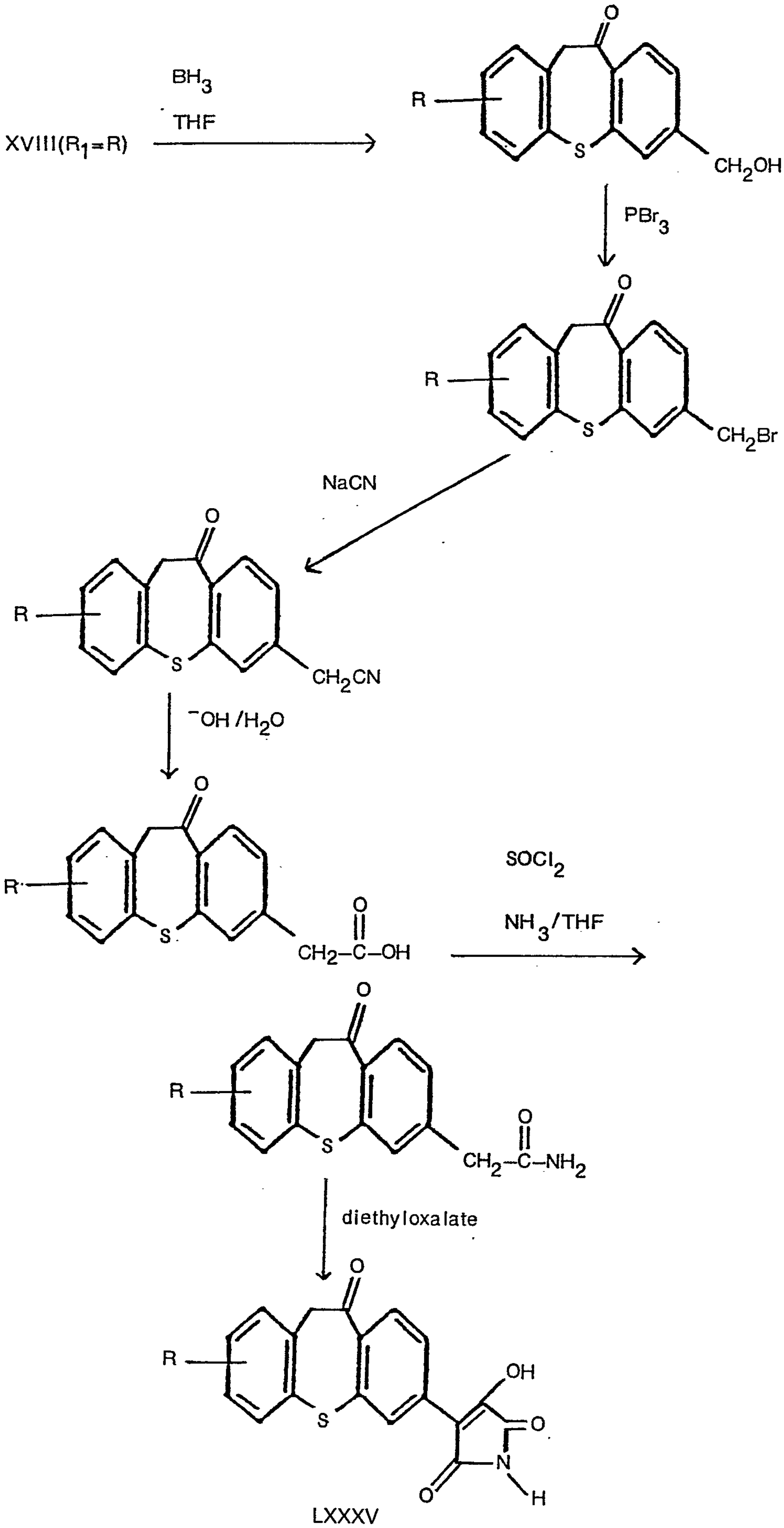
XVIII($R_1$=R)
$BH_3$
THF
$PBr_3$
NaCN
$^-OH$ / $H_2O$
$SOCl_2$
$NH_3$/THF
diethyloxalate

LXXXV

Where corresponding sulfinyl or sulfonyl derivatives are desired, the products of the four reaction schemes described immediately above may be oxidized by the techniques already described.

It will be noted that the reaction sequence described above affords not only thiepins of this invention wherein the substituent at the 3-position is 4-hydroxy-$\Delta^3$-pyrroline-3-yl-2,5-dione, but, in Steps A—D, leads also to the preparation of those thiepins of this invention wherein the substituent at the 3-position is a loweralkanoic acid (i.e., compounds of formula I wherein A is

$$-\overset{\overset{\displaystyle O}{\|}}{C}R_2,$$

n is an integer between 1 and 4 and $R_2$ is hydroxy). Thus, Steps A—D, as described above, starting with the appropriately substituted 3-carboxylic acid, through reduction, bromination, cyanization and oxidation, affords the corresponding 3-acetic acid derivative. Quite obviously, the described reduction, bromination, cyanization and oxidation sequence can be repeated, employing the 3-acetic acid derivative as starting material, in order to obtain the corresponding propionic acid derivative which, in turn, can be employed as starting material for preparing the corresponding butyric acid derivative. In this manner, any desired 3-loweralkanoic acid derivative of the instant invention readily is prepared. Corresponding sulfinyl or sulfonyl derivatives are prepared by the oxidation techniques previously described.

The 3-cyanoloweralkyl intermediates obtained from Steps A—C in the reaction sequence described above also serve as intermediates in the preparation of other therapeutically active thiepins of formula I. Thus, for example, an appropriately substituted 3-cyanomethyl-10,11-oxodibenzo[b,f]thiepin may be treated with sodium azide and ammonia by techniques previously described to form the corresponding 3-(1H-tetrazol-5-ylmethyl)-10,11-dihydro-11-oxodibenzo[b,f]thiepin and the product, if desired, can be oxidized to form the corresponding sulfinyl or sulfonyl derivative.

As noted above, pharmaceutically acceptable salts of the novel thiepins also are included within the scope of this invention. The term, pharmaceutically acceptable salts, is intended to include salts derived from pharmaceutically acceptable non-toxic acids and bases such as, for example, ammonium salts, alkali metal salts such as sodium and potassium salts, alkaline earth metal salts such as magnesium and calcium salts, salts of organic bases such as amine salts derived from mono-, di and tri-loweralkyl or loweralkanoyl amines such as trimethylamine, dimethylamine and triethanolamine, salts derived from heterocyclic amines such as piperidine, pyridine, piperazine and morpholine, and salts derived from pharmaceuticaly acceptable acids such as hydrochloric acid, sulfuric acid, tartaric acid and propionic acid.

Example 1

Step 1

2-(3-Bromophenylthio)benzoic Acid

A mixture consisting of 179 g. m-dibromobenzene (0.758 mole); 46.6 g. thiosalicyclic acid (0.303 mole); 25.9 g cuprous oxide (0.181 mole); 212 cc. quinoline; and 24 cc. pyridine is mechanically stirred and heated in an oil bath at 200°C. to 210°C. for three hours. The internal temperature remains constant at 145°C. The reaction mixture is then poured into 1500 cc. of 5N aqueous HCl. The oily solid is filtered; then dissolved in 750 cc. 1N aqueous NaOH solution; this solution is filtered through celite, then extracted three times with ether. The aqueous fraction is acidified with concentrated HCl and the resulting grayish solid filtered, washed well with water and dried. The crude yield obtained is 65 g. (69.4%). It is used as such in the next step.

Step 2

2-(3-Bromophenylthio)benzyl Alcohol

A solution of 63.3 g. of 2-(3-bromophenylthio)benzoic acid (0.205 mole, crude) in 400 cc. dry tetrahydrofuran (THF) is treated dropwise at room temperature and under nitrogen atmosphere with 240 cc. 0.96N borane in THF. Hydrogen evolution is noticed during approximately one-third of the addition. After completion of the addition, the mixture is left stirring for one additional hour, then decomposed by the dropwise addition of 15 cc. water. Most of the THF is evaporated off and the residue is partitioned ether and water. The organic phase contains the crude alcohol which is chromatographed on a column of silica gel (1 kg.); elution is done with a mixture of 20% ethyl acetate in benzene. Pure 2-(3-bromophenylthio)-3-nitrobenzyl alcohol (50.03 g.) is obtained as a yellow oil (82.8% yield).

Step 3

2-(3-Bromophenylthio)benzyl Bromide

To 50.03 g. 2-(3-bromophenylthio)benzyl alcohol (0.17 mole) cooled in an ice bath is added dropwise 6 cc. phosphorous tribromide (0.06 mole). The resulting milky oil is stirred in the cold an

additional 15 minutes then decomposed with ice water. Extraction with ether affords 58.6 g. of 2-(3-bromophenylthio)benzyl bromide as a yellowish-brown oil which is used as such in the next step.

Step 4
2-(3-Bromophenylthio)benzyl Cyanide

12.4 g sodium cyanide (0.25 mole) is added to a solution of 58.6 g. 2-(3-bromophenylthio)benzyl bromide (0.164 mole) in 200 cc. dimethylformamide (DMF). The reaction is slightly exothermic. The resulting solution is allowed to stir for an hour without cooling, then is diluted with a large volume of water and extracted with ether three times. Ether extracts are washed several times with water, dried over sodium sulfate and the ether evaporated off. The crude yield of 2-(3-bromophenylthio)benzyl cyanide is 49.6 g.; the product is an oil which is hydrolyzed as such.

Step 5
2-(3-Bromophenylthio)phenyl Acetic Acid

49.6 g crude 2-(3-bromophenylthio)benzyl cyanide is refluxed in a mixture of 650 cc. 20% aqueous sodium hydroxide solution and 650 cc. denatured alcohol for three hours. The solution is concentrated and the sodium salt of the acid separates. The mixture is diluted to three liters with water and acidified with concentrated HCl. The free acid precipitates, and is filtered. The yield of crude 2-(3-bromophenylthio)phenyl acetic acid is 49.3 g. (93.5%). It is used as such in the next step.

Step 6
3-Bromo-10,11-dihydro-11-oxodibenzo[b,f]thiepin

49.3 g (0.1526 mole) crude 2-(3-bromophenylthio)phenyl acetic acid and 50 cc. thionyl chloride are refluxed together for 10 minutes. The excess thionyl chloride is evaporated off and the residual oil dissolved in 1,2-dichloroethane and the mixture evaporated again to remove the last traces of thionyl chloride.

The oily acid chloride is dissolved in 400 cc. 1,2-dichloroethane and 22.4 g. aluminum chloride (10% excess) is added in portions. The reaction is slightly exothermic but no cooling is necessary. The reaction is permitted to go for 40 minutes then the mixture is poured onto ice. The organic fraction is collected and the aqueous fraction extracted three times with chloroform. Combined organics are washed with water, dried over sodium sulfate and stripped to dryness. The solid residue is triturated in ether and filtered, then triturated in methanol and filtered. The yield of crude product is 34.8 g. (74.8%).

Step 7
3-Cyano-10,11-dihydro-11-oxodibenzo[b,f]thiepin

36.45 g of 3-bromo-10,11-dihydro-11-oxodibenzo[b,f]thiepin and 32.11 g cuprous cyanide are refluxed in 450 cc dimethylformamide for $5\frac{1}{2}$ hours. The reaction mixture is cooled down and poured into 2 l. of ice water with good mechanical stirring. The resulting solid is filtered, washed with water and then extracted into 1.7 l. chloroform. The insoluble copper salts are filtered and the filtrate evaporated to dryness. The residue is triturated in methanol and the insoluble yellow product is filtered and weighs 22.4 g (74.6%), m.p. 166°—170°C.

Step 8
3-Cyano-10,11-dihydro-11-oxodibenzo[b,f]thiepin-5-oxide

1.63 g of 3-cyano-10,11-dihydro-11-oxodibenzo[b,f]thiepin is dissolved in 100 cc methylene chloride. 1.12 g *m*-chloroperbenzoic acid is added and the solution is stirred for one hour at room temperature. The reaction mixture is diluted with methylene chloride, excess of powdered calcium hydroxide is added, and the mixture is stirred for five minutes and filtered through Celite. The organic filtrate is evaporated to dryness and the residue chromatographed on silica gel and eluted with a 1:1 mixture of benzene:chloroform to yield 937 mg (54%) of the desired pure sulfoxide, m.p. 219°—221°C.

Step 9
10,11-Dihydro-11-oxodibenzo[b,f]thiepin-3-carboxylic acid 5-oxide

780 mg of 3-cyano-10,11-dihydro-11-oxodibenzo[b,f]thiepin-5-oxide is refluxed for four hours in a mixture of 50 cc. 10% aqueous sodium hydroxide and 50 cc. ethanol. The reaction mixture is diluted with water, acidified with hydrochloric acid, filtered and dried under vacuum in the oven at 70°C. The solid is recrystallized from a mixture of dimethylformamide and methanol to yield 360 mg. of the pure acid, m.p. 273°C. dec.

Calculated: C: 62.93; H: 3.52; S: 11.20.
Found: C: 62.86; H: 3.77; S: 11.13.

Example 2

10,11-Dihydro-11-oxo-3-(5-tetrazolyl)dibenzo[b,f]thiepin

To 25 cc. of freshly distilled tetrahydrofuran is added in the following order 2.35 g. aluminum chloride, 2 g. 3-cyano-10,11-dihydro-11-oxodibenzo[b,f]thiepin (see Example 1 Steps 1 to 7 for its preparation), and 2.3 g. sodium azide, and the mixture is refluxed for 8 hours, cooled and 7 cc. of 15% aqueous hydrochloric acid added. The supernatant layer is decanted and the residue triturated with ethyl acetate. The combined organic phases are extracted with aqueous sodium bicarbonate solution. The basic aqueous solution is acidified with hydrochloric acid and the solid filtered. After two recrystallizations from methanol, 850 mg. (36%) of the pure tetrazole is obtained, m.p. 235°C. dec.

Calculated: C: 61.21; H: 3.42; N: 19.03; S: 10.89.

Found: C: 60.92; H: 3.60; N: 18.76; S: 10.63.

Example 3

Methyl 10,11-Dihydro-11-oxodibenzo[b,f]thiepin-3-carboxylate

Step 1

10,11-Dihydro-11-oxodibenzo[b,f]thiepin-3-carboxylic acid

Reflux 20 g of 3-cyano-10,11-dihydro-11-oxodibenzo[b,f]thiepin, whose preparation is described in Example 1 Steps 1 to 7, in a mixture of 200 cc concentrated hydrochloric acid and 200 cc acetic acid for 21 hours. The desired acid is precipitated out of the solution. Filter it off while hot, wash the residue with water and dry it to yield 17.3 g of light-yellow solid. From the mother liquors 2.74 g of additional material is recovered, total yield 93.6%.

Step 2

3-Chlorocarbonyl-10,11-oxodibenzo[b,f]thiepin-3-carboxylate

Dissolve 5.16 g of 10,11-dihydro-11-oxodibenzo[b,f]thiepin-3-carboxylic acid in 100 cc of chloroform and 50 cc of thionyl chloride and add to the mixture 1.0 cc of dimethylformamide. Allow the mixture to stand at room temperature for 72 hours. Evaporate the mixture to dryness to obtain the desired acid chloride.

Step 3

Methyl 10,11-dihydro-11-oxodibenzo[b,f]thiepin-3-carboxylate

Dissolve 2.0 g of the acid chloride of Step 2 in 20 cc of tetrahydrofuran containing 1.0 cc of methanol and 4 cc of pyridine. Allow the mixture to stand at room temperature for 24 hours, then evaporate to dryness. Dissolve the residue in 1:4 ether/hexane and filter through silica gel. Evaporate the filtrate to dryness to obtain the title product.

By the process of Example 3 but substituting another lower alkanol, for example, ethanol, 2-propanol, butanol or 2-butanol, for the methanol of Step 2, the corresponding lower alkyl esters of 10,11-dihydro-11-oxodibenzo[b,f]thiepin-3-carboxylic acid are obtained.

Example 4

10,11-Dihydro-11-oxodibenzo[b,f]thiepin-3-carboxamide

Step 1

3-Chlorocarbonyl-10,11-dihydro-11-oxodibenzo[b,f]thiepin

Heat a solution of 5 g of 10,11-dihydro-11-oxodibenzo[b,f]thiepin-3-carboxylic acid (see Example 3, Step 1) and 40 ml of thionyl chloride under reflux for 20 minutes. Evaporate the reaction mixture under vacuum to dryness. Repeat the evaporation with two 30 ml portions of carbon tetrachloride. Crystallize the residue from diisopropyl ether to obtain the title product.

Step 2

10,11-Dihydro-11-oxodibenzo[b,f]thiepin-3-carboxamide

Dissolve the acid chloride from Step 1 in 20 ml of dry tetrahydrofuran and add this solution dropwise with stirring to a cooled (ice-bath) saturated solution of ammonia in 60 ml of tetrahydrofuran. Pass ammonia through the reaction for 15 minutes. Stir at room temperature for an additional 15 minutes and evaporate the reaction mixture to dryness. Add a mixture of 12 ml of ethanol and 60 ml of water to the residue and stir at room temperature for an additional 30 minutes. Separate the solid by filtration and wash with water, then with ethanol and then with ether. Dry *in vacuo* to obtain the title product.

Example 5

10,11-Dihydro-11-oxodibenzo[b,f]thiepin-3N-methylcarboxamide

Add 6.0 g of 3-chlorocarbonyl-10,11-dihydro-11-oxodibenzo[b,f]thiepin (see Example 3, Step 2) to 4 g of methylamine in 100 ml of methylene chloride at 0—5°C. Add 13 ml of triethylamine dropwise over 10 minutes, then stir the reaction mixture at room temperature overnight. Extract the reaction mixture with water, dry the organic layer and evaporate to dryness. Chromatograph over silica gel,

eluting with 200:20 toluene/dioxane. Evaporate the eluate to dryness and recrystallize the residue from methanol to obtain the title compound.

In a similar manner, substituting another N-loweralkylamine, for example, ethylamine, propylamine, isopropylamine or butylamine, or a N,N-diloweralkylamine, for example, dimethylamine, diethylamine, dipropylamine or dibutylamine, for the methylamine, there is obtained the corresponding 10,11-dihydro-11-oxodibenzo[b,f]thiepin-3-N-lower alkylcarboxamide or 3-N,N-diloweralkylcarboxamide.

Also in a similar manner, substituting a carboxyloweralkylamine, for example, glycine, valine, leucine or isoleucine and the like or a N-(lower alkyl)derivative thereof, for example, N-methylglycine, N-propylleucine or N-butylisoleucine, there is obtained the corresponding 10,11-dihydro-11-oxodibenzo[b,f]thiepin-3-carboxyloweralkyl carboxamide or a N-(lower alkyl) derivative thereof.

Example 6

10,11-Dihydro-11-oxodibenzo[b,f]thiepin-3-N-methanesulfonylcarboxamide

Heat 5.0 g of 10,11-dihydro-11-oxodibenzo[b,f]thiepin-3-carboxylic acid (see Example 3, Step 1) in 50 cc of thionyl chloride for 15 minutes at reflux and then distil off the excess of thionyl chloride. Evaporate the residue twice with small volumes of benzene. Add the resulting acid chloride to 4.0 g of methane sulphonamide in 100 ml of methylene chloride at 0—5°C. Add dropwise over 10 minutes 15 ml of triethylamine. Stir the mixture at room temperature overnight. Extract the reaction mixture with 100 cc of 0.5 *N* sodium hydroxide, wash the alkaline extract with ether and acidify with 6*N* hydrochloric acid. Separate the solids by filtration and dry *in vacuo* over potassium hydroxide. Chromatograph over silica gel eluting with 200:20:3 toluene/dioxane/acetic acid. Evaporate the eluate to dryness and recrystallize the residue from methanol to obtain the title product.

In a similar manner, substituting another loweralkylsulphonamide, for example, ethanesulphonamide, propane sulphonamide or butane sulphonamide, for the methane sulphonamide, there is obtained the corresponding 10,11-dihydro-11-oxodibenzo[b,f]thiepin-2-N-loweralkylsulfonylcarboxamide.

Example 7

10,11-Dihydro-11-oxodibenzo[b,f]thiepin-3-(3-methyl-2-thiazolidinylidene)carboxamide

Reflux 1.0 g of 10,11-dihydro-11-oxodibenzo[b,f]thiepin-3-carboxylic acid (see Example 3, Step 1) in 15 cc of thionyl chloride for 30 minutes. Strip the reaction mixture to dryness and dissolve the residue in 25 cc of methylene chloride. Add a solution of 1.0 g of 2-imino-3-methylthiazolidine in 10 cc of methylene chloride. Stir at room temperature for 30 minutes and add water. Continue stirring for 10 minutes. Separate the organic phase, wash it with water and dry it overnight over sodium sulfate. Strip to dryness. Stir and triturate the residue in ether, then in methanol. Chromatograph the resulting solid over silica gel, eluting with 20% ethylacetate in benzene. Strip to dryness to obtain the title product.

Example 8

10,11-Dihydro-11-oxodibenzo[b,f]thiepin-3-(4-hydroxy-$\Delta^3$-pyrroline-3-yl-2,5-dione)

Step 1

3-Hydroxymethyl-10,11-dihydro-11-oxodibenzo[b,f]thiepin

Dissolve 5.1 g of 10,11-dihydro-11-oxodibenzo[b,f]thiepin-3-carboxylic acid (see Example 3, Step 1) in 100 cc of tetrahydrofuran and add 35 cc of 1M borane in tetrahydrofuran at room temperature under a nitrogen atmosphere. Stir the mixture at room temperature for 3 hours. Slowly dilute the reaction mixture with water and then with ethyl acetate. Wash it with aqueous sodium chloride, dry and evaporate to an oil.

Step 2

3-Bromomethyl-10,11-dihydro-11-oxodibenzo[b,f]thiepin

Dissolve 4.43 g of the alcohol of Step 1 in 100 cc of benzene and add 1 cc (10.5 millimoles) of phosphorus tribromide. Stir at room temperature for 1 hour, add water and then dilute with toluene. Wash three times with water, dry and strip to a solid residue.

Step 3

3-Cyanomethyl-10,11-dihydro-11-oxodibenzo[b,f]thiepin

Dissolve 6.4 g of the bromide of Step 2 in 75 cc. of dimethylformamide and add 2.95 g of sodium cyanide. Stir the mixture at room temperature for 1.5 hours. Dilute with 600 cc. of water and extract three times with ether. Wash the combined organics with water, dry and strip to a solid residue. Triturate in hexane and recover the solid by filtration.

Step 4

10,11-Dihydro-11-oxodibenzo[b,f]thiepin-3-acetic acid

Reflux 2.0 g of the nitrile of Step 3 in a mixture of 30 cc. of 20% aqueous sodium hydroxide and

30 cc. of ethanol for four hours. Strip away the alcohol, wash with ethyl acetate and acidify the aqueous phase with hydrochloric acid. Separate the precipitate by filtration. Wash with water and dry.

Step 5
10,11-Dihydro-11-oxodibenzo[b,f]thiepin-3-acetamide

Reflux for 20 minutes a mixture of 5.0 g of the acid of Step 4 and 40 ml. of thionyl chloride. Evaporate to dryness under vacuum. Evaporate twice with 30 ml. portions of carbon tetrachloride. Dissolve the residue in 20 ml. of tetrahydrofuran and add the solution dropwise to a cooled and stirred saturated solution (ice bath) of ammonia in 60 ml. of tetrahydrofuran. Pass ammonia through the solution simultaneously. Continue stirring at room temperature for an additional 15 minutes. Evaporate the mixture to dryness. Add a mixture of 12 ml. of ethanol and 60 ml. of water and stir the suspension for 30 minutes. Separate the solids and wash with water, then with ethanol and finally with ether to obtain the title product.

Step 6
10,11-Dihydro-11-oxodibenzo[b,f]thiepin-3-(-4-hydroxy-$\Delta^3$-pyrrolin-3-yl-2,5-dione)

Stir at room temperature a mixture of 5.118 gm. of the amide of Step 5, 2.939 gm. of diethyloxalate, 4.723 gm. of potassium t-butoxide and 40 ml. of dimethylformamide for 6 hours. Pour the reaction mixture into 300 ml. of ice-water and extract with 300 ml. of ethyl acetate. Acidify with 6N hydrochloric acid and separate the ethyl acetate layer. Wash with saturated sodium chloride solution and dry. Evaporate to dryness and dissolve the residue in warm dioxane. Treat with a slight excess of ammonia and separate the solid by filtration. Wash with dioxane and dry. Suspend the product in water, acidify with 6N hydrochloric acid and extract with ethyl acetate. Wash the extract with saturated sodium chloride solution, dry over magnesium sulfate and evaporate to obtain the title product.

Example 9

$\beta$-Hydroxyethyl 10,11-Dihydro-11-oxodibenzo[b,f]thiepin-3-carboxylate

To a stirred solution of 1.0 g of 3-chlorocarbonyl-10,11-dihydro-11-oxodibenzo[b,f]thiepin (see Example 3, Step 2) in 50 cc of methylene chloride, add 3 g of ethylene glycol and stir the mixture for 18 hours at room temperature. Distil off the solvent and excess of ethylene glycol under high vacuum (0.1 mm). Chromatograph the residue on a silica gel column (100 g), eluting with 10% ethyl acetate in benzene to obtain the title product.

In a similar manner, substituting another loweralkyldiol such as, for example, trimethylene glycol or 1,4-butanediol for the ethylene glycol, there is obtained the corresponding hydroxyloweralkylester.

Example 10

$\beta$-Dimethylaminoethyl-10,11-Dihydro-11-oxodibenzo[b,f]thiepin-3-carboxylate

Dissolve 1.0 g of 3-chlorocarbonyl-10,11-dihydro-11-oxodibenzo[b,f]thiepin as prepared in Example 3, Step 2, in 10 cc of anhydrous tetrahydrofuran with stirring and add 2 ml of N,N-dimethylethanolamine. Stir at room temperature for 18 hours and strip the mixture to dryness. Partition the residue between ether and dilute hydrochloric acid and separate the aqueous layer. Basify the aqueous layer with aqueous ammonia and extract with ethyl acetate. Evaporate the organic phase and chromatograph the residue over silica-gel eluting with 90% chloroform in methanol to obtain the title product.

In a similar manner, substituting another N,N-diloweralkylaminoloweralkanol, for example, diethylethanolamine, 3-N,N-dimethylaminopropan-1-ol or 4-N,N-diethylaminobutan-1-ol, for the N,N-dimethylethanolamine, there is obtained the corresponding N,N-diloweralkylaminoloweralkyl ester.

Example 11

10,11-Dihydro-11-oxodibenzo[b,f]thiepin-3-N-carboxymethylcarboxamide

Reflux 1.0 g of 3-chlorocarbonyl-10,11-dihydro-11-oxodibenzo[b,f]thiepin in 20 cc of ethyl acetate containing 2.0 g of glycine for 5 hours. Evaporate the mixture to dryness. Add 30 cc of water to the solid residue and stir at room temperature for one hour. Separate the solid by filtration and recrystallize from ethanol to obtain the title product.

In a similar manner, substituting another amino acid, for example, alanine or valine, for the glycine, there is obtained the corresponding 3-carboxyloweralkylcarboxamide.

Example 12

$\beta$-Carboxyethyl 10,11-Dihydro-11-oxodibenzo[b,f]thiepin-3-carboxylate

Dissolve 1.0 g of 3-chlorocarbonyl-10,11-dihydro-11-oxodibenzo[b,f]thiepin in 20 cc of tetrahydrofuran and add 1.0 g of the sodium salt of $\beta$-hydroxypropionic acid. Stir the mixture at room temperature for 18 hours. Filter and evaporate the filtrate to dryness. Recrystallize the solid residue from ethanol to obtain the title product.

In a similar manner, substituting another hydroxyloweralkanoic acid salt, for example, an alkali metal salt of hydroxyacetic acid or 3-hydroxypropionic acid sodium salt, there is obtained the corresponding carboxyloweralkyl-3-carboxylate ester.

Example 13

3-(3-Hydroxy-1,2,5-thiadiazol-4-yl)-10,11-dihydro-11-oxo-dibenzo[b,f]thiepin

Step 1

3-Cyano-10,11-dihydro-11-oxodibenzo[b,f]thiepin

Stir 5 g of methyl 10,11-dihydro-11-oxodibenzo[b,f]thiepin-3-carboxylate (see Example 3 Step 3) in 500 ml of methanol saturated with ammonia gas for 24 hours at room temperature. Evaporate the reaction mixture to dryness. Reflux the residue in 200 ml of methylene chloride containing 10 g of phosphorus oxychloride for eight hours. Cool the reaction mixture to room temperature and shake several times with water. Separate the organic layer, dry over magnesium sulfate and evaporate it to dryness to obtain the title product.

Step 2

10,11-Dihydro-11-oxodibenzo[b,f]thiepin-3-carboxaldehyde

Heat a mixture of 5.0 g of 3-cyano-10,11-dihydro-11-oxodibenzo[b,f]thiepin and 4.0 g of Raney nickel alloy in 60 ml of 75% (v/v) aqueous formic acid at reflux for $1\frac{1}{2}$ hours. Cool to room temperature and filter. Concentrate to small volume and extract with methylene chloride. Wash the extract with water and with 1N sodium bicarbonate until neutral. Dry the neutral extract over sodium sulfate and concentrate it to dryness to obtain the title product.

Step 3

10,11-Dihydro-11-oxodibenzo[b,f]thiepin-3-(2-aminoacetonitrile)

Stir at room temperature for 12 hours a mixture of 5.85 g of ammonium chloride, 5.3 g of sodium cyanide, 75 ml. of ammonium hydroxide, 100 ml. of ethanol saturated with ammonia and 12 gm. of carboxaldehyde of Step 1. Pour the reaction mixture into 300 ml. of water and extract with ether. Dry the extract over sodium sulfate and concentrate to dryness to obtain the title product.

Step 4

10,11-Dihydro-11-oxodibenzo[b,f]thiepin-3-(2-aminoacetamide)

Stir at room temperature 5.0 g of the aminoacetonitrile of Step 2 in 30 ml. of concentrated hydrochloric acid for 30 minutes. Slowly pour the reaction mixture into cold ammonium hydroxide. Extract the mixture with ether and dry over sodium sulfate. Evaporate the extract to dryness to obtain the title product.

Step 5

3-(3-Hydroxy-1,2,5-thiadiazol-4-yl)-10,11-dihydro-11-oxodibenzo[b,f]thiepin

Stir overnight at room temperature a mixture of 1.365 g of the aminoacetamide of Step 4, 1.989 g of sulfur monochloride and 5 ml. of dimethylformamide. Filter the reaction mixture and then partition between ice-water (75 ml.) and ethyl acetate (75 ml.). Filter, separate the organic layer, wash with saturated aqueous sodium chloride solution and dry over magnesium sulfate. Evaporate to dryness and dissolve the residue in 200 ml of boiling ethanol, treat with charcoal and filter. Concentrate to 25 ml and separate the solids by filtration to obtain the title product.

Example 14

3-(1H-Tetrazol-5-ylmethyl)-10,11-dihydro-11-oxodibenzo[b,f]thiepin

Add to 25 cc of tetrahydrofuran cooled in an ice bath 1.59 g (11.9 millimoles) of aluminium chloride, 1.33 g (5.25 millimoles) of 3-cyanomethyl-10,11-dihydro-11-oxodibenzo[b,f]thiepin (see example 8 Step 3) and 1.55 g (23.8 millimoles) of sodium azide. Reflux the mixture into ethyl acetate and evaporate. Triturate the residue in ether and separate the title product by filtration.

Example 15

10,11-Dihydro-11-oxodibenzo[b,f]thiepin-3-acetic acid-5,5-dioxide

Heat 600 mg of 10,11-dihydro-11-oxidibenzo[b,f]thiepin-3-acetic acid (see Example 8 Step 4) to 80—85°C in a mixture of 30 cc of glacial acetic acid and 5 cc of 30% hydrogen peroxide for 3 hours. Dilute with water to a final volume of about 250 cc. Separate the title product by filtration.

The compounds of formula I are useful in the treatment or prophylaxis of mammalian disease conditions where excessive undesirable contractile activity of prostaglandins, such as $PGF_{2a}$, or prostaglandin biosynthetic intermediates contribute. These conditions include asthma, inflammatory states such as arthritis, allergy, diarrhea, hypertension, angina, platelet aggregation, cerebral spasm, premature abortion and dismenorrhea. In particular, they are of value in reaginic mediated asthma (extrinsic asthma).

The magnitude of a prophylactic or therapeutic dose of compound of formula I will, of course, vary with the nature and the severity of the condition to be treated and with the particular compound of formula I and its route of administration. In general, the dose range lies within the range of 0.2 mg to 100 mg. per kg. body weight of a mammal.

The pharmaceutical compositions of the present invention comprise a compound of formula I as an active ingredient, and may also contain pharmaceutically acceptable carrier and optionally other therapeutic ingredients. The compositions include compositions suitable for oral, rectal, opthalmic, pulmonary, nasal, dermal, topical or parenteral (including subcutaneous, intramuscular and intravenous) administration, although the most suitable route in any given case will depend on the nature and severity of the condition being treated and on the nature of the active ingredient. They may be conveniently presented in unit dosage form and prepared by any of the methods well known in the art of pharmacy.

For use where a composition for intravenous administration is employed, a suitable dosage range is from 0.2 to 10 mg. (preferably 1 to 5 mg.) of a compound of formula I per kg. of body weight per day and in the case where an oral composition is employed a suitable dosage range is about, e.g., 1 to 50 mg. of a compound of formula I per kg. of body weight per day, preferably from 10 to 40 mg./kg.

Pharmaceutical compositions of the present invention suitable for oral administration and by inhalation in the case of asthma therapy may be presented as discrete units such as capsules, cachets or tablets each containing a predetermined amount of the active ingredient; as a powder or granules; or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface active or dispersing agent. Moulded tablets may be made by moulding in a suitable machine, a mixture of the powdered compound moistened with an inert liquid diluent. Desirably, each tablet contains from 50 mg. to 500 mg. of the active ingredient and each cachet or capsule contains from 50 mg. to 500 mg. of the active ingredient.

Although the instant invention has been described in the foregoing specification in terms of the use of the novel thiepin disclosed herein in the treatment and control of human and warm-blooded animal disease conditions characterized by excessive undesirable contractile activity of prostaglandins and prostaglandin biosynthetic intermediates, and particularly of asthma, it will be recognized by those skilled in the art that, in addition to the involvement of contractile prostaglandins in chronic obstructive lung disease (e.g., asthma), prostaglandins play a role in other allergic conditions as well as in inflammation, diarrhea, hypertension, angina, cerebral spasm, premature abortion and dismenorrhea. Also, the thiepins of this invention are potent $TXA_2$ biosynthesis inhibitors, inhibiting platelate aggregation, and can be useful in diseases such as atherosclerosis, variant anginal and myocardial infarction.

**Claims**

1. A compound of the formula:

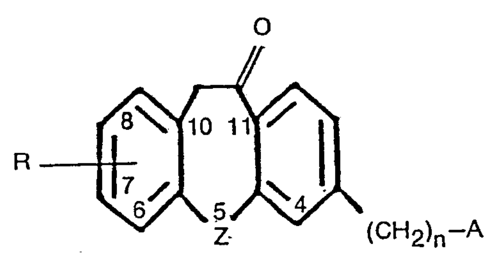

I

in which *n* is 0 or an integer from 1 to 4; Z is thio, sulfinyl, or sulfonyl; R is in the 7-position or the 8-position and is hydrogen, chlorine, bromine, fluorine, iodine, amino, $C_{1-4}$ alkyl, $C_{1-4}$ alkanoyl, hydroxyl, $C_{1-4}$ alkoxy, mercapto, $C_{1-4}$ alkylthio, $C_{1-4}$ alkylsulfinyl, $C_{1-4}$ alkylsulfonyl, trifluoromethyl, trifluoromethylthio, cyano, carboxy, nitro, $C_{1-4}$ alkylamino or di($C_{1-4}$ alkyl)amino; A is 5-tetrazolyl, 3-hydroxy-1,2,5-thiadiazol-4-yl, 4-hydroxy-2,5-dioxo-$\Delta^3$-pyrrolin-3-yl or

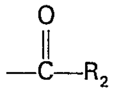

where $R_2$ is hydroxy, $C_{1-4}$ alkoxy, N,N-di($C_{1-4}$ alkyl)amino-($C_{1-4}$ alkoxy), $C_{1-4}$ hydroxyalkoxy, carboxy-($C_{1-4}$ alkoxy), amino, $C_{1-4}$ alkylamino, di($C_{1-4}$ alkyl)amino, $C_{1-4}$ alkylsulfonylamino, carboxy($C_{1-4}$ alkyl)-amino, carbamoyl($C_{1-4}$ alkyl)amino or 2-imino-3-methylthiazolidine with the proviso that $R_2$ is not hydroxy when *n* is 0, R is hydrogen and Z is thio; and the pharmaceutically acceptable salts thereof.

2. A compound according to claim 1 in which *n* is 0 and A is 5-tetrazolyl.

3. A compound according to claim 1 in which *n* is 1, 2, 3 or 4 and A is carboxy.

4. A compound according to any one of claims 1 to 3 in which R is chloro.

5. A compound according to any one of claims 1 to 3 in which R is bromo.

6. 10,11-Dihydro-11-oxodibenzo[b,f]thiepin-3-carboxylic acid-5-oxide.

7. 10,11-Dihydro-11-oxo-3-(5-tetrazolyl)dibenzo[b,f]thiepin.

8. A process for preparing a compound of the formula:

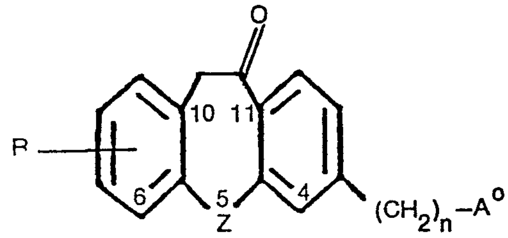

in which *n*, Z and R are as defined in claim 1 and A° is 5-tetrazolyl or carboxy that comprises hydrolysing a compound of the formula:

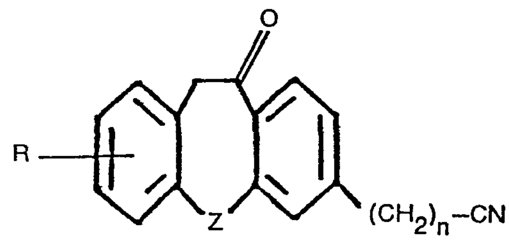

with an acid or base to form the carboxyl compound or reacting the said compound with an azide ion to form the 5-tetrazolyl compound.

9. A composition for treating undesirable contractile activity of prostaglandin comprising a therapeutically effective amount of a compound as claimed in any one of claims 1 to 7.

**Patentansprüche**

1. Verbindung der Formel:

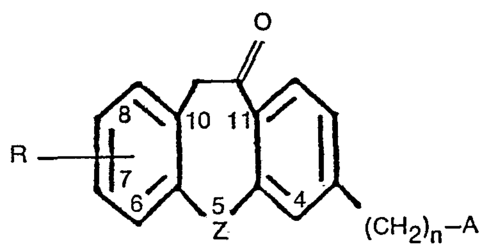

I

in welcher bedeuten: *n* 0 oder eine Zahl von 1 bis 4; Z Thio, Sulfinyl oder Sulfonyl; R, welches sich in der 7-Stellung oder 8-Stellung befindet, Wasserstoff, Chlor, Brom, Fluor, Jod, Amino, $C_{1-4}$-Alkyl, $C_{1-4}$-Alkanoyl, Hydroxyl, $C_{1-4}$-Alkoxy, Mercapto, $C_{1-4}$-Alkylthio, $C_{1-4}$-Alkylsulfinyl, $C_{1-4}$-Alkylsulfonyl, Trifluoromethyl, Trifluoromethylthio, Cyano, Carboxy, Nitro, $C_{1-4}$-Alkylamino oder Di-($C_{1-4}$-alkyl)-amino; A 5-Tetrazolyl, 3-Hydroxy-1,2,5-thiadiazol-4-yl, 4-Hydroxy-2,5-dioxo-$\Delta^3$-pyrrolin-3-yl oder

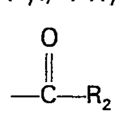

wobei $R_2$ bedeutet: Hydroxy, $C_{1-4}$-Alkoxy, N,N-Di($C_{1-4}$-alkyl)-amino-($C_{1-4}$-Alkoxy), $C_{1-4}$-Hydroxyalkoxy, Carboxy($C_{1-4}$-alkoxy), Amino, $C_{1-4}$-Alkylamino, Di-($C_{1-4}$-alkyl)amino, $C_{1-4}$-Alkylsulfonylamino, Carboxy-($C_{1-4}$-alkyl)amino, Carbamoyl-($C_{1-4}$-alkyl)-amino oder 2-Imino-3-methylthiazolidin, mit der Massgabe, dass $R_2$ nicht Wasserstoff ist, wenn *n* 0, R Wasserstoff und Z Thio bedeuten; und deren pharmazeutisch annehmbare Salze.

2. Verbindung nach Anspruch 1, in welcher *n* 0 und A 5-Tetrazolyl bedeuten.
3. Verbindung nach Anspruch 1, in welcher *n* 1, 2, 3 oder 4 und A Carboxy bedeuten.
4. Verbindung nach irgendeinem der Ansprüche 1 bis 3, in welcher R Chloro bedeutet.
5. Verbindung nach irgendeinem der Ansprüche 1 bis 3, in welcher R Bromo bedeutet.
6. 10,11-Dihydro-11-oxodibenzo[b,f]thiepin-3-carbonsäure-5-oxid.
7. 10,11-Dihydro-11-oxo-3-(5-tetrazolyl)-dibenzo[b,f]thiepin.
8. Verfahren zur Herstellung einer Verbindung der Formel:

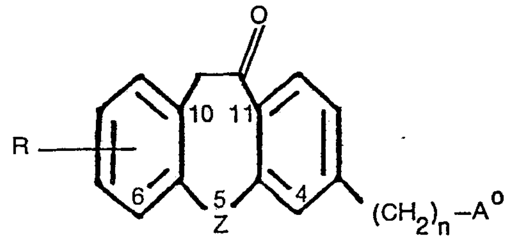

in welcher *n*, Z und R wie in Anspruch 1 definiert sind und A° 5-Tetrazolyl oder Carboxy bedeutet, welches die Schritte umfasst: Hydrolysieren einer Verbindung der Formel:

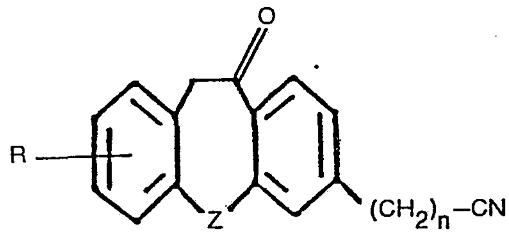

mit einer Säure oder einer Base, um die Carboxylverbindung zu bilden, oder Umsetzen der genannten Verbindung mit einem Azidion, um die 5-Tetrazolylverbindung zu bilden.

9. Zusammensetzung zur Behandlung unerwünschter Kontraktionswirksamkeit von Prostaglandin, umfassend eine therapeutisch wirksame Menge einer Verbindung nach irgendeinem der Ansprüche 1 bis 7.

## Revendications

1. Composé de la formule:

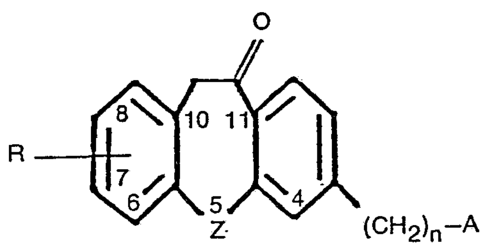

I

dans laquelle *n* est 0 ou un nombre entier de 1 à 4; Z est un groupe thio, sulfinyle ou sulfonyle; R se trouve dans la position 7 ou dans la position 8 et est de l'hydrogène, du chlore, du brome, du fluor, de l'iode, un groupe amino, alcoyle en $C_{1-4}$, alcanoyle en $C_{1-4}$, hydroxyle, alcoxy en $C_{1-4}$, mercapto, (alcoyl en $C_{1-4}$)thio, (alcoyl en $C_{1-4}$)-sulfinyle, (alcoyl en $C_{1-4}$)sulfonyle, trifluorométhyle, trifluorométhylthio, cyano, carboxy, nitro, (alcoyl en $C_{1-4}$)amino ou di(alcoyl en $C_{1-4}$)amino; A est un groupe 5-tétrazolyle, 3-hydroxy-1,2,5-thiadiazol-4-yle, 4-hydroxy-2,5-dioxo-$\Delta^3$-pyrrolin-3-yle ou

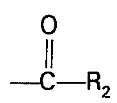

où $R_2$ est un groupe hydroxy, alcoxy en $C_{1-4}$, N,N-di(alcoyl en $C_{1-4}$)amino-(alcoxy en $C_{1-4}$), hydroxyalcoxy en $C_{1-4}$, carboxy-(alcoxy en $C_{1-4}$), amino, (alcoyl en $C_{1-4}$)amino, di(alcoyl en $C_{1-4}$)amino, (alcoyl en $C_{1-4}$)sulfonylamino, carboxy(alcoyl en $C_{1-4}$)amino, carbamoyl(alcoyl en $C_{1-4}$)amino ou 2-imino-3-méthylthiadiazoline avec la condition que $R_2$ n'est pas un groupe hydroxy quand *n* est 0, R est de l'hydrogène et Z est un groupe thio; et ses sels pharmaceutiquement acceptables.

2. Composé selon la revendication 1, caractérisé en ce que *n* est 0 et A est un groupe 5-tétrazolyle.

3. Composé selon la revendication 1, caractérisé en ce que *n* est 1, 2, 3 ou 4 et A est un groupe carboxy.

4. Composé selon l'une quelconque des revendications 1 à 3, caractérisé en ce que R est du chlore.

5. Composé selon l'une quelconque des revendications 1 à 3, caractérisé en ce que R est du brome.

6. 5-oxyde d'acide 10,11-dihydro-11-oxodibenzo(b,f]thiépine-3-carboxylique.

7. 10,11-dihydro-11-oxo-3-(5-tétrazolyl)dibenzo(b,f)thiépine.

8. Procédé pour la préparation d'un composé de la formule:

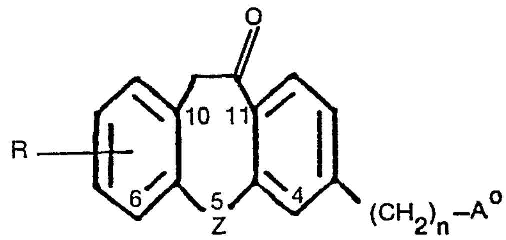

dans laquelle *n*, Z et R sont tels que définis dans la revendication 1 et A° est un groupe 5-tétrazolyle ou carboxy, caractérisé en ce qu'on hydrolyse un composé de la formule:

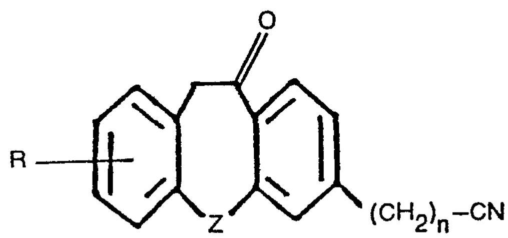

avec un acide ou une base de manière à former le composé carboxylé ou on fait réagir ce composé avec un ion azide de manière à former le composé 5-tétrazolyle.

9. Composition pour le traitement de l'activité contractile indésirable de prostaglandines, comprenant une quantité thérapeutiquement efficace d'un composé selon l'une quelconque des revendications 1 à 7.